# EUROPEAN PATENT APPLICATION

(11) **EP 4 566 534 A1**
(43) Date of publication of application: **11.06.2025**
(21) Application number: 24217984.4
(22) Date of filing: 06.12.2024
(51) Int. Cl.: A61B 5/145, A61B 5/00

(54) **MICRONEEDLE ARRAY AND METHOD FOR PRODUCING A MICRONEEDLE ARRAY**

(30) Priority: 07.12.2023 LU 505713
(71) Applicant: Christian-Albrechts-Universität zu Kiel - Körperschaft des öffentlichen Rechts, 24118 Kiel (DE)
(72) Inventor: Zeynep, Altintas, 21449 Kiel (DE); Sharifuzzaman, Md, 24143 Kiel (DE)
(74) Representative: Günther, Constantin

(57) **Abstract**

The invention is related to a microneedle array comprising a substrate and a plurality of hydrogel-forming microneedles arranged in the form of one or more arrays. The invention is also related to a method for producing such a microneedle array. The hydrogel-forming microneedles may be coated with a conductive polymer layer of PEDOT:PSS. The resulting microneedles may have an electrical conductivity of 100 S/m or more. Laser-based photothermal techniques such as laser-scribed phase separation (LSPS) may be used for enhancing the electrical conductivity and in vivo water stability of the PEDOT:PSS coating layer.

## Description

The invention is related to a microneedle array comprising a substrate and a plurality of microneedles arranged in the form of one or more arrays. The invention is also related to a method for producing such a microneedle array.

Microneedle arrays can be used for sensing physiological markers such as glucose in the interstitial fluid of human tissues, as described in CN 114748064 A.

Transdermal or Interstitial fluid (ISF) is formed from blood in capillaries and contains 83% of the same components as plasmas. It forms the basis of wound exudate when it leaks into a wound cavity. The inflammatory process triggered by the formation of a wound releases a variety of substances (mediators and enzymes) that, among other effects, promote the formation of wound exudate by increasing ISF productions. Finding a potential correlation between wound ISF and wound bed exudate could be a game-changing advance in the management of chronic wounds. Microneedle (MN) patches have recently demonstrated significant promise in the development of multifunctional dressings for efficient drug delivery in chronic wound therapy. Hydrogel-forming MNs (HFMNs) are an alternative to solid MN because they extract transdermal fluid or ISF into their swellable porous matrix, are capable of wound healing, and can maintain the wound environment's hydration level. Despite these unique advantages, HFMNs have not yet been utilized for in-vivo and real-time chronic wound theranostics (monitoring and healing) due to their limited conductivity, stability, biocompatibility, sensing, and self-therapeutic issues.

It is an object of the invention to improve such microneedle array and its methods of use, as well as providing for an improved method for producing such microneedle array.

The object of the invention is achieved by a microneedle array comprising a substrate and a plurality of microneedles arranged in the form of one or more arrays as a dressing system, and comprising at least two or at least six sensors configured for sensing wound-specific bio-indicators of a living being wearing the microneedle array, wherein the microneedles are configured as hydrogel-forming microneedles with high electric conductivity and water stability suitable for conducting electrical signals from the living being to the sensors. The microneedle array of the invention allows for a wider area of use cases in the diagnosis and/or therapy of living beings, in particular for a personalized wound management. The same hydrogel-forming microneedles offer noninvasive and on-site healing capabilities due to the anti-inflammatory and antimicrobial properties of the hydrogel. The microneedle array can be applied to any kind of living being (human or animal).

Therefore, the invention applies a dual functionality of the same microneedles for both real-time diagnosis of the wound and for therapeutic treatment of the wound. The materials used in the microneedle array are designed such that they do not inhibit biological growth on the sensors. The sensors can be integrated directly onto the microneedles. The sensors are configured for real-time electrochemical readout (amperometric and voltametric) of chronic wound-specific biomarkers in transdermal wound fluid. The on-needle sensing mechanism allows for immediate detection and processing of biochemical signals directly at the wound site, facilitating rapid and responsive treatment actions.

The microneedle array may comprise at least six microneedle sensors and one needle-free sensor, all configured for onsite transdermal and epidermal sensing of specific bio-indicators from a living being, facilitating simultaneous modulation of wound healing.

The microneedles are designed to be conductive and maintain stability in water, enabling them to effectively transmit electrical signals from the living being to the sensors, broadening the scope for diagnosing and treating individuals, particularly in personalized wound care settings. These hydrogel-forming microneedles also offer noninvasive healing benefits due to their anti-inflammatory and antimicrobial properties and are applicable to various living beings, including humans and animals.

Normally, the use of hydrogel-forming microneedles leads to a very low electrical conductivity, which is not suitable for conducting electrical signals from the living being to the sensors. However, in the present invention the electric conductivity is increased such that the hydrogel-forming microneedles can be used for conducting electrical signals from the living being to the sensors. For example, the electric conductivity of the hydrogel-forming microneedles can be at least 100 S/m or at least 200 S/m or at least 300 S/m or at least 384 S/m (Siemens per meter).

The microneedle array has microneedles which have dimensions in the micrometer range. The length of a microneedle is less than 1000 µm. The microneedles can be conically-shaped. As far as such microneedles are arranged in the form of an array, this includes a regular array, like a matrix form, or any kind of irregular array. In an advantageous embodiment of the invention, the size and in particular the length of the microneedles can be chosen dependent on the pathology of the area where the microneedle array shall be applied.

According to an advantageous embodiment of the invention, the microneedle array is configured for diagnosis and/or therapy of the chronic wound of the living being. Such chronic wounds are typically wounds which do not heal or heal very slowly, e.g. in the case of a Decubitus. For example, the invention includes configurations for the microneedle array to function in diagnosing and treating chronic wounds at the transdermal level. These wounds, which heal slowly, like decubitus ulcers, benefit from this targeted approach. Further enhancements include photothermal techniques to modify the microneedles, significantly increasing their electrical conductivity and water stability

According to an advantageous embodiment of the invention, the microneedle array is configured for diagnosis and/or therapy of the chronic wound in dermis and/or epidermis level of the living being. This allows for a very wide-spread and extended application of the microneedle array of the invention.

According to an advantageous embodiment of the invention, the microneedle array or at least the microneedles are created and/or modified by photothermal techniques separately, e.g. by a CO₂ laser-based photothermal process like laser-scribed phase separation (LSPS). In particular, through such a photothermal process the electrical conductivity and water stability of the microneedles can be increased, even though they are established as hydrogel-forming microneedles.

According to an advantageous embodiment of the invention, the microneedle array is configured for measuring at least one parameter from the wound surface to which the microneedle array is applied to, in order to assess exudate characteristics. This enables correlation with transdermal wound fluid analyses performed by the microneedle arrays to which it is applied, enhancing diagnostic precision. In this way, problems of healing of the chronic wound can be better identified and measures for healing the chronic wound can be more precisely applied.

For example, the microneedle array can be configured for measuring at least one parameter of the transdermal wound fluid or interstitial fluid (ISF) to get information about exudate of the wound to which the microneedle array is applied to. In this way, problems of healing of the chronic wound can be better identified and measures for healing the chronic wound can be more precisely applied.

According to an advantageous embodiment of the invention, the microneedle array is configured for minimal invasive implantation in the dermis and/or epidermis level of the skin of a living being. This allows for a very simple application of the microneedle array to a patient.

According to an advantageous embodiment of the invention, the microneedles are arranged in separate groups (insulas) on the substrate, wherein each group is separated from other groups by an area of the substrate surrounding the group and having no microneedles. In this way, several sensor areas can be established on the substrate. For example, each group of microneedles can be arranged on the substrate in a circular shape. It is also possible to arrange an outer ring of microneedles which surrounds the separate groups of microneedle arrays. According to an advantageous embodiment, each of the sensors can be implemented in one of the groups of microneedles.

The sensors of the microneedle array can be of different kind, for measuring different kinds of physical and/or biochemical markers of the living being simultaneously. The sensors of the microneedle array can be biosensors of any type. In this way, through the microneedle array of the invention different indicators of the living being can be measured at the same time. In particular, the microneedle array can have different types of sensors for sensing different kinds of biomarkers, e.g. sensors for glucose (Glu), uric acid (UA), pH, Na⁺, Cl⁻, K⁺, and/or temperature (T). For example, the microneedle array can have at least three sensors or at least four sensors.

According to an advantageous embodiment of the invention, the substrate is an elastic substrate, e.g. a flat substrate. This has the advantage that the microneedle array can be produced in an uniform shape and can be applied to different parts of the body of the living being. By the elasticity of the substrate the microneedle array has the capability to adopt its shape, at least in a certain range, to the shape of the part of the body where it is applied to.

According to an advantageous embodiment of the invention, the microneedle array or at least the microneedles are coated with a functionalizing material and thereby functionalized. For example, the functionalizing material can provide for better electrical conductivity of the microneedles. It is also possible that the functionalizing material can be a therapeutic material, e.g. a pharmaceutical substance, which supports healing of the chronic wound.

According to an advantageous embodiment of the invention, one, more or all of the sensors of the microneedle array can be replaced on the substrate. In this way, one or more of the sensors can be exchanged or replaced using the same substrate. Such replaceable sensors within the microneedle array allow for a long-term use, and providing for better maintenance and adaptability to different wound types. The substrate may comprise a mounting place for each exchangeable sensor. For example, the microneedle array can be removed from a living being, then one or more sensors are replaced by other kinds of sensors or by new sensors, and then the microneedle array can be attached again to a living being.

The object of the invention is also achieved by a system for sensing wound-specific bio-indicators of a living, comprising a microneedle array of the aforementioned kind and an electronic circuit which is electrically connected to the sensors of the microneedle array, wherein the electronic circuit is configured for evaluating, storing and/or displaying the bio-indicators sensed by the sensors.

The object of the invention is also achieved by a method for producing a microneedle array (1) or at least the microneedles are created and/or modified by photothermal techniques separately, e.g. by a CO₂ laser-based photothermal process like LSPS, wherein the electrical conductivity of the hydrogel-forming microneedles is increased by the photothermal process. In this way, the electrical conductivity of the hydrogel-forming microneedles can be increased efficiently.

According to an advantageous embodiment of the invention, by the photothermal process, the base material of the sensor is coated with a functionalizing material and thereby functionalized. For example, the functionalizing material can provide for better electrical conductivity of the microneedles. It is also possible that the functionalizing material can be a therapeutic material, e.g. a pharmaceutical substance, which supports healing of the chronic wound.

The invention is also related for a method of diagnosis and/or therapy of a chronic wound of a living being, wherein a microneedle array of the aforementioned kind is applied to the chronic wound and used for diagnosis and/or therapy of the chronic wound.

In this way the invention can provide a fully-integrated conductive hydrogel-forming multiplexed implantable microneedle patch for chronic disease diagnosis and therapy. The several aspects of the invention are hereinafter further explained.

Chronic diseases, including chronic wounds (CWs), referred to as non-healing wounds, can be caused by diabetes, decubitus ulcers, venous dysfunction, aging and surgery. At each stage of CWs, the chemical composition of the wound changes significantly, indicating the stage of wound healing and even the presence of infection. Personalized wound management requires both efficient wound care and close monitoring of vital wound healing biomarkers. Recently, numerous wearable wound monitoring devices have been applied to better understand the wound environment in real time by gaining insights from wound environmental markers. Although wearable technologies have opened up exciting-opportunities¹⁻⁴, they still face significant obstacles before they can fully fulfill their potential for useful in vivo chronic wound management applications.

The present invention overcomes disadvantages of the prior art by:
i) The microneedle array and/or its biosensors and/or their therapeutic systems do not have to rely on stratum corneum-level wound exudate. Wounds that are shallow, flat and dried do not exude wound exudate. Therefore, collection of necessary wound exudate is a difficult task for wearable wound management systems.
ii) The microneedle array is designed in a way that it overcomes requirement for permeability and biocompatibility of wearable devices to protect the wound bed from bacterial infiltration and infection and regulate wound exudate levels.
iii) The microneedle array is able to deliver moisture and/or rehydrate necrotic tissue and can provide chemical therapies.
iv) The microneedle array is a stable and dynamic system. It provides for miniaturized user-interactive theranostic systems and can therefore by easily commercialized and practically used in in-vivo applications.

In the present invention, we innovate a fully integrated and highly conductive smart system based on HFMNs that can accommodate multiple independent electrochemical and physiological sensors on a single small array to efficiently verify the conditions of CWs by multiplexed and multimodal wound biomarker analysis in the transdermal wound fluid or ISF as well as in the wound bed and to present a possible correlation between both biofluids. In the broadest sense, a group of biomarkers consisting of Glu, UA, pH, Na⁺, Cl⁻, K⁺, and T may be selected based on their relevance to the infectious, metabolic and inflammatory state of chronic wounds as well as other chronic diseases.

Biocompatible and/or swellable polymer - e.g. polyvinyl alcohol (PVA), polycaprolactone (PCL), hyaluronic acid, and/or polylactic-co-glycolic acid (PLGA) with embedded conductive MXenes nanofillers (e.g. Ti₃C₂Tₓ, V₂CTₓ, Mo₂CTₓ) may be used to construct the sensor platforms of the HFMN device. MXene has metallic conductivity and a larger specific surface area as well as a proven electronegative functional group (-OH, -F, -O). The introduction of electronegative groups onto MXene triggers the formation of three-dimensional polymer hydrogel networks via hydrogen (H) bonds. This mechanism improves the hydrogel's mechanical and electrical properties. In addition, MXene plays an important role in the healing of CWs due to the antimicrobial activity of its surface functional groups. The hydrogel inhibits wound infection and promotes skin wound healing by stimulating NIH-3T3 proliferation.

In order to generate conductive electrochemical sensor patterns on to hydrogel, conductive polymer mixture, comprising either Polyaniline (PANI), Polythiophene (PTh), or Polyacetylene (PA), combined with Poly(3-hexylthiophene) (P3HT) or poly(3,4-ethylenedioxythiophene): polystyrene sulfate (PEDOT: PSS), further integrated with light-responsive two-dimensional materials selected from black phosphorus (BP), molybdenum disulfide (MoS₂), and Graphene oxide (GO). This configuration enables the activation of the microneedle array by photothermal techniques using a smart control system that dynamically adjusts laser parameters to optimize the photothermal energy and electric field applied to the PANI or PTh or PA/P3HT or PEDOT: PSS/BP or MoS₂ or GO coating, thereby enhancing the electrical conductivity and in vivo water stability are significantly improved by the larger and interconnected PEDOT-rich domains created by the photothermal techniques. Inventively, the photothermal technique integrates multiple fabrication processes into a single electrode preparation step. With the potential correlation between ISF and exudate, this system bridges the gap between conventional wearables and microneedles. It is expected to be a game changer in the clinical management of chronic diseases and their commercialization.

The invention comprises a novel strategy for the fabrication of a highly conductive and biocompatible hydrogel-forming implantable microneedle-based dressing system for chronic wounds diagnosis and therapy. The implantable system can be simply placed on the patient's wound and secured to the wound area e.g., using a Tegaderm transparent film dressing (3M). After that, the system can record data from wound-based transdermal interstitial fluid (ISF), and the patient can monitor seven wound-specific indicators, e.g., Glu, UA, pH, Na⁺, Cl⁻, K⁺, and T, based on their significance in reflecting the infection, metabolic, and inflammatory status of the CWs. Furthermore, the hydrogel inhibits wound infection and promotes cutaneous wound healing by stimulating NIH-3T3 cell proliferation due to its antimicrobial properties. This combined diagnostic and prognostic tool will ensure better and more precise clinical management of the patient and their wounds.

The novel custom-engineered, fully integrated implantable dressing could serve as a more effective, fully controllable, and easy-to-implement platform for personalized monitoring and treatment of CWs with minimal side effects. The system does not depend on surface wound fluid, compared with conventional wearable dressings.

The system proposed here can be mass-produced and cheap, offers a versatile platform for evaluating wound conditions and intelligent therapy, and can be easily reconfigured to monitor several other metabolic and inflammatory biomarkers for various chronic disease applications.

The microneedle array comprises minimally invasive, highly conductive hydrogel-forming microneedles (HFMNs)-based multimodal implantable dressing system that continuously monitors the on-site physiological conditions of chronic wounds (CWs) and offers noninvasive healing capabilities due to the anti-inflammatory and antimicrobial properties of the hydrogel. The unique sensor array design enables the integration of multiple (e.g. seven) replaceable sensors in the form of HFMNs sensing electrodes to target the desired wound-specific analytes in transdermal interstitial fluid (ISF) (e.g. Glu, UA, pH, Na⁺, Cl⁻, K⁺, and T) based on their significance in reflecting the infection, metabolic, and inflammatory status of the CWs.

The hydrogel may be composed of a biocompatible and swellable polymer - e.g. polyvinyl alcohol (PVA), polycaprolactone (PCL), hyaluronic acid, and polylactic-co-glycolic acid (PLGA) and crosslinked with, and chitosan or gelatin as a crosslinking agent, while the incorporation of MXenes (Ti₃C₂Tₓ, V₂CTₓ, Mo₂CTₓ) nanosheets as conductive nanofillers facilitates the formation of 3D polymer hydrogel networks via hydrogen (H) bonding

Further coating and functionalization of conductive polymer mixture, comprising one or more of poly(3,4-ethylenedioxythiophene): polystyrene sulfate (PEDOT: PSS), graphene oxide (GO), Polyaniline (PANI), Polythiophene (PTh), or Polyacetylene (PA), combined with Poly(3-hexylthiophene) (P3HT), further integrated with light-responsive two-dimensional materials selected from black phosphorus (BP), molybdenum disulfide (MoS₂), and Graphene oxide (GO). This configuration enables the activation of the microneedle array by photothermal techniques using a smart control system that dynamically adjusts laser parameters to optimize the photothermal energy and electric field applied to the PANI or PTh or PA/P3HT or PEDOT: PSS/BP or MoS₂ or GO coating, thereby enhancing the electrical properties (100 S/m or at least 200 S/m or at least 300 S/m or at least 384 S/m) necessary to transduce electrochemical signals. Inventively, the photothermal technique integrates multiple fabrication processes into a single electrode preparation step and facilitates the mass production of highly conductive HFMNs-based electrodes for the first time. Through the potential correlation between wound-affected ISF and wound bed exudate, this system bridges the gap between conventional CWs electrodes and implantable dressing systems. It is anticipated to be a game-changing advancement in the clinical management of chronic diseases and their commercialization.

In the following the invention is further describes by examples using drawings. The drawings show:
- Figure 1: The fabrication concept of an HFMNs-based implantable dressing system for CWs diagnostics and therapy,
- Figure 2: Physical characterizations of the HFMNs,
- Figure 3: Design and characterization of the sensor array for multiplexed wound analysis in ISF,
- Figure 4: Correlation of the HFMNs system and conventional sensors,
- Figure 5: Antimicrobial activity of the hydrogel,
- Figure 6: A system with a microneedle array,
- Figure 7: Another fabrication concept of an HFMNs-based implantable dressing system for CWs diagnostics and therapy,
- Figure 8: Physical characterizations of the HFMNs,
- Figure 9: Comparative electrochemical response of microneedles to glucose with and without the laser-based photothermal effect,
- Figure 10: Correlation of the HFMNs system and conventional sensors,
- Figure 11: Antimicrobial activity of the hydrogel,
- Figure 12: Cell viability of fibroblasts.

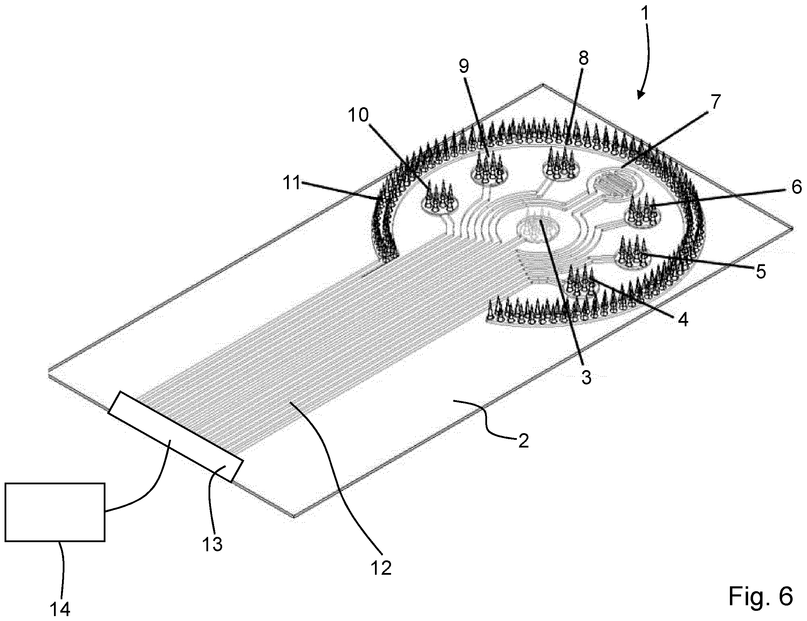

First, a system for sensing wound-specific bio-indicators of a living being is described with reference to Figure 6. As shown, the system for comprises a microneedle array 1 and an electronic circuit 14. The microneedle array 1, which is in the following also referred to a HFMNs-based implantable dressing system, has a plurality of sensors 4, 5, 6, 7, 8, 9, 10 for sensing different bio-indicators. Sensors 4, 5, 6, 8, 9, 10 are implemented in insular-like, separated groups of microneedles. The microneedles of each group might be arranged in the form of an array with a round outer shape. Sensor 7 does not have microneedles, it can be for example a T (temperature) sensor or other electrochemical sensor. Further, in the center of the sensors 4, 5, 6, 7, 8, 9, 10 a reference electrode 3 is placed which is similarly constructed as the sensors 4, 5, 6, 8, 9, 10, namely having an array of microneedles. In addition, the sensors 4, 5, 6, 7, 8, 9, 10 can be surrounded by a circular stripe of microneedles which establish a counter electrode 11 of the arrangement. The sensors 4, 5, 6, 7, 8, 9, 10 as well as the reference electrode 3 and the counter electrode 11 can be applied on the surface of a common substrate 2. The sensors 4, 5, 6, 7, 8, 9, 10 and the electrodes 3, 11 can be connected through wires 12 to a plug-in connector 13 for connecting an electronic evaluation circuit 14. However, the electronic circuit 14 can also be placed on the substrate 2. The electronic evaluation circuit 14 can evaluate the electrochemical and physical signals provided by sensors 4, 5, 6, 7, 8, 9, 10.

### Design and fabrication strategies of the fully-integrated HFMNs-based implantable dressing system

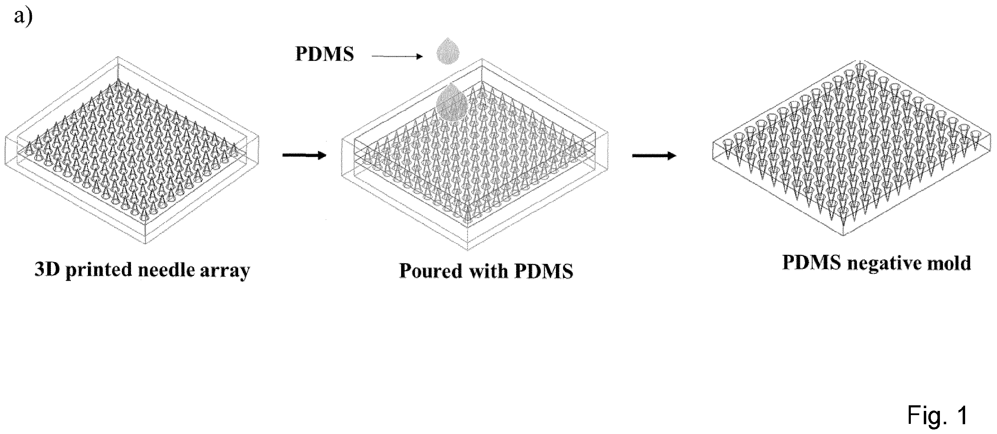

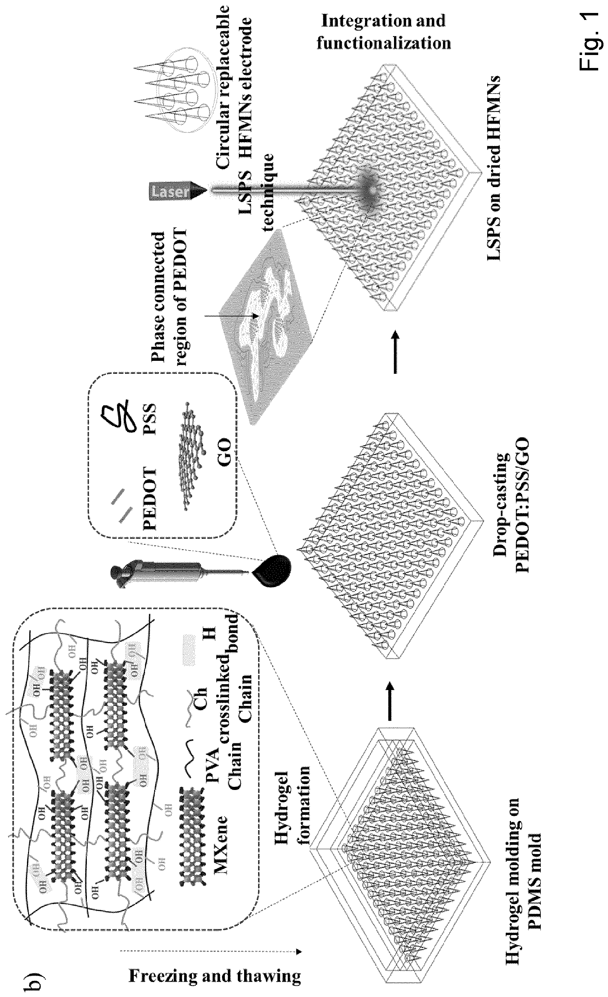

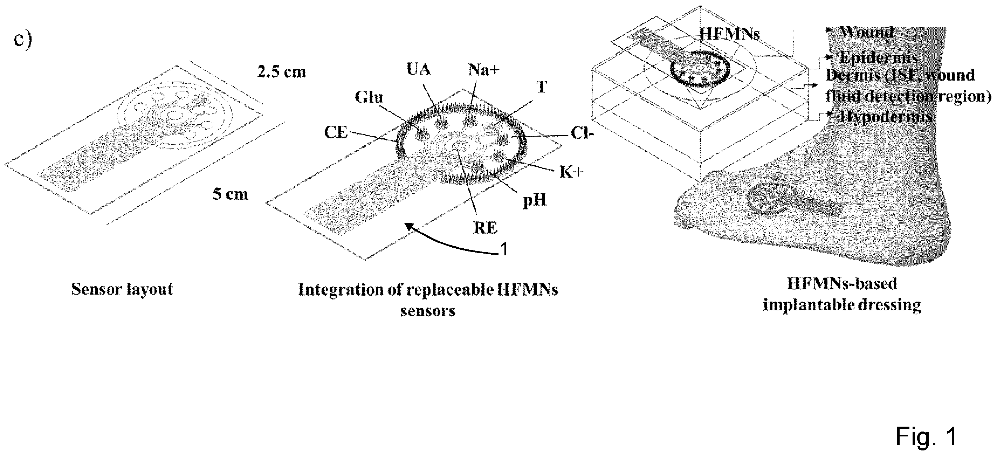

Figure 1 shows the fabrication concept of an HFMNs-based implantable dressing system for CWs diagnostics and therapy. The fabrication process in step a) involves designing the HFMNs and their 3D printing. Step b) includes Hydrogel formation, coating of PEDOT: PSS/GO, and subsequent LSPS process, Step c) includes sensor layout (CE-counter electrode and RE-reference electrode), integration and functionalization of the replaceable sensors onto the sensor pad, and placement of the HFMN-based implantable system for CWs monitoring and therapy.

Herein, solid microneedles consisting of 49×43 conically-shaped needles (800 µm in height) arranged atop a 60×40 mm film (5mm in thickness) were produced via stereolithography 3D printing (Figure 1a, 1st step) using a computer-aided design (CAD) and used as the master mold for the generation of polydimethylsiloxane (PDMS) negative molds (Figure 1b, 2nd and 3rd step), followed by hydrogel casting on the PDMS molds (Figure 1b, 1st step). After two times of freezing and thawing, the HFMNs were then released from PDMS, dried under vacuum, and utilized for further modifications. Opting to use 3D printing for microneedle development can provide a scalable, consistently reproducible, and significantly cheaper manufacturing approach. The hydrogel was made of polyvinyl alcohol (PVA), chitosan (Ch), and MXene (Ti₃C₂Tₓ), in which the biocompatible PVA can form a 3D network with Ch via cross-linking by repeated freezing and thawing (Figure 1 b, 1st step), and Ch can improve the porosity and water absorption capacity of hydrogel⁷. The phase transition property and 3D network structure of the PVA/Ch hydrogel make the microneedle suitable for both easy penetration into skin and the extraction of sufficient ISF. MXene has metallic conductivity and a larger specific surface area, as well as an improved electronegative functional group (-OH, -F, -O)⁸. In the presence of electronegative groups on Ti₃C₂Tₓ, 3D polymer hydrogel networks form through the hydrogen (H) bonds and enhance the mechanical and electrical properties of the hydrogel (see the hydrogel formation in Figure 1b, 1st step inset). A 360° electroactive layer of PEDOT: PSS and graphene oxide (GO) was deposited onto the 3D-printed microneedles via dropcasting onto both sides of the microneedles (Figure 1b, 2nd step). Following successive washings and thorough drying, the microneedle-PEDOT: PSS-coated composites were prepared for the next step.

### Laser-scribed phase separation (LSPS)

PEDOT: PSS exhibits a phase dispersion in which the insulating PSS is located in the shell and the conductive PEDOT is located in the core. The PSS shell around the PEDOT core not only obstructs charge flow but also plays a role in PEDOT dispersion⁹. A key approach for converting PEDOT: PSS into hydrogels that are water-stable is to restructure the phase organization of the material. Phase separation is a novel method for producing conductive PEDOT: PSS hydrogels by careful alteration of the orientation and crystallization of the PEDOT and PSS-rich regions, respectively^{10,11}. Precisely, outstanding electrical conductivity and aqueous stability can be achieved through a robust interconnection between the hydrophobic and conductive regions rich in PEDOT. Therefore, we employed the novel laser patterning process to fabricate highly conductive hydrogels via the LSPS without the need for a separate postchemical cleaning step on PEDOT: PSS/GO coated HFMNs (Figure 1b, 3rd step). In order to optimize laser absorption, PEDOT: PSS was sonicated with GO solution with varying concentrations as the solvent. This process facilitated the separation and recrystallization of PEDOT and PSS via a fast temperature increase and an enhanced electric field. We proposed that any laser is capable of simultaneously supplying photothermal energy and an electric field to PEDOT: PSS/GO, in light of the improved properties of PEDOT: PSS-based hydrogels.

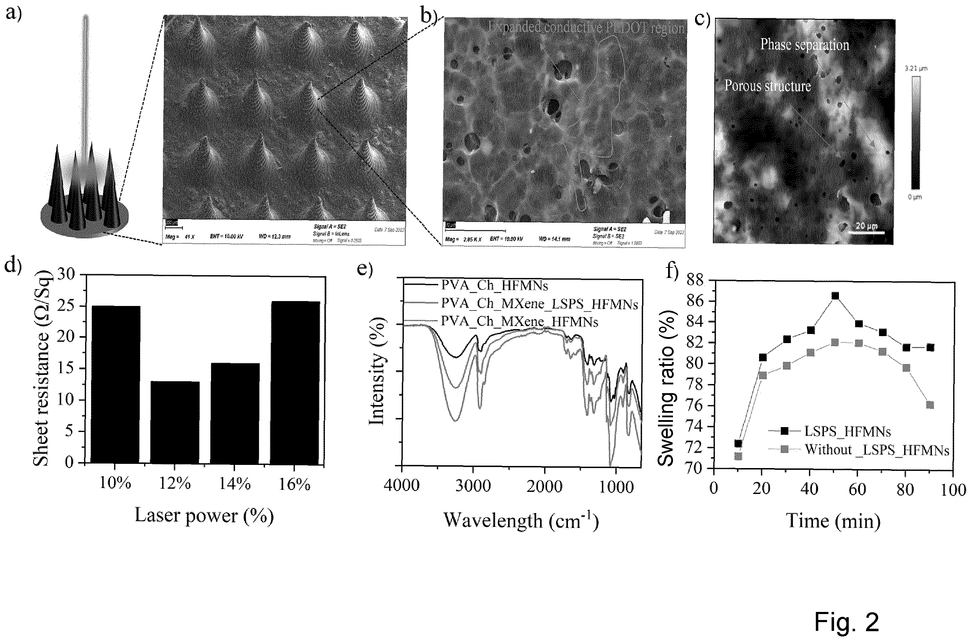

Figure 2 shows physical characterizations of the HFMNs. a) SEM image of the laser-scribed HFMNs and b) expanded porous PEDOT rich region due to LSPS. c) AFM phase image of LSPS. d) Laser power optimization for LSPS. e) FTIR spectra analysis. f) Swelling ratio.

As shown by scanning electron microscopy (SEM) (Figure 2a), HFMNs exhibited a conical structure with a length of -P800 µm, a sharp tip of -P10 µm, and inter-microneedle spacing of 700 µm after the LSPS technique. This emphasizes the homogenous microstructural characteristics of the coated laser-scribed microneedles. Selective phase separation by the laser was confirmed with SEM and atomic force microscopy (AFM) phase image analysis (Figure 2b and 2c). The PEDOT-rich domain was greatly expanded (bright color) and connected after the LSPS. When the laser was working properly, increasing the laser power (LP) made PEDOT: PSS more electrically conductive and stable in water. But when the laser power was too high, PEDOT: PSS turned into carbon and its conductivity started to drop (Figure 2d). For LP 12%, combined with the effect of GO, fast laser scanning (300 s) can achieve the highest electrical conductivity (sheet resistance of 13 Ω/Sq). After the LSPS, the desired circular-shape electrodes can be cut and utilized for replaceable sensors for CWs monitoring and treatment in the dermis and/or epidermis level (Figure 1c, 2nd and 3rd steps). This novel manufacturing process ensures the fabrication and mass production of conductive HFMNs at a fast-processing speed compared with other processes.

The Fourier transform infrared (FT-IR) spectra of the PVA/Ch, PVA/Ch/MXene, and LSPS-based PVA/Ch/MXene HFMNs (Figure 2e) showed peaks at 3417, 2927, 1616, 1413, and 1029 cm⁻¹, which correspond to the O-H stretching vibration, C-H stretching vibration, COO-stretching vibration, carbonyl (C=O) stretching vibration, and C-O-C stretching vibration¹². By comparison, it seems that after LSPS, the peak intensity of the HFMNs decreased slightly due to the increase in hydrophobic PEDOT-rich regions.

As shown in Figure 2f, the swelling rate and water content of the HFMNs and LSPS-based HFMNs were compared. The results showed that the LSPS technique increased its swelling rate and water stability. The increased swelling rate can make the gel more effective at absorbing ISF and wound healing.

### Design and characterization of the HFMNs sensor array for multiplexed CW-biomarker analysis

For unavoidable inactivation of enzymes and mechanical friction, we utilized a replaceable sensor component as an effective strategy to prolong the service life of the implantable systems. A unique polar-type sensor array of 7 working electrodes was fabricated on a flexible substrate (2.5×5 cm) with their leads insulated with a waterproof layer of PDMS (Figure 1c, 1st step). All active circular laser-cutter HFMNs-based sensors (Glu, UA, pH, Na⁺, Cl⁻, K⁺, and T) along with the counter electrode (CE) and reference electrode (RE) are placed in independent and replaceable modules (Figure 1c, 2nd step). Therefore, if an electrode is used, expired, or damaged, a replacement "insert" can simply be dropped into place in the sensor platform. Moreover, it will be easier to switch the production line from one analyte to the next in a manufacturing environment. The electrode pad was brush-painted with a thin layer of conductive Ag paste as the liquid adhesive and then coupled with a patch of five 2 × 2 arrays of HFMNs. The performance of the sensor was characterized by artificial ISF. For the first time, we developed this novel strategy for the fabrication of HFMNs-based implantable dressing systems. The concentrations of key markers in wound exudate (WX) and ISF could generally be considered as (Glu: ISF (0-7) mM and WX (0-28) mM, UA: ISF (200-600) µM and WX (0-760) µM, Na⁺ and CI-: ISF: (0.75-200) mM and WX (5-160) mM, K⁺: ISF (1-128) mM and WX (3.2-5.7) mM, pH: ISF (5-9) and WX (4-9), and T: (25-40) °C ^{13,14.} Therefore, the concentration range of the corresponding markers in ISF is larger than the wound exudate range, which pose another major challenge to obtain linear sensor response in the physiological concentration ranges. To address these issues and achieve accurate wound fluid metabolic monitoring, increase sensor range, and minimize biofouling effects, we explored the use novel functionalization process and an outer porous membrane that serves as a diffusion limiting layer to protect the enzyme, tune response, increase operational stability, as well as enhance the linearity and sensitivity magnitude of the sensor. We fabricated our enzymatic Glu and UA sensor based on Platinum nanoparticles/ EDC-NHS/Glucose oxidase or Uricase (Pt NPs/ EDC-NHS /GOx or UOx) with additional porous membrane coatings with polyurethane (PU). The triple coating of PU-based enzymatic sensors showed the highest linearity over the wide physiological concentration range as well as high reproducibility in complex wound fluid matrix.

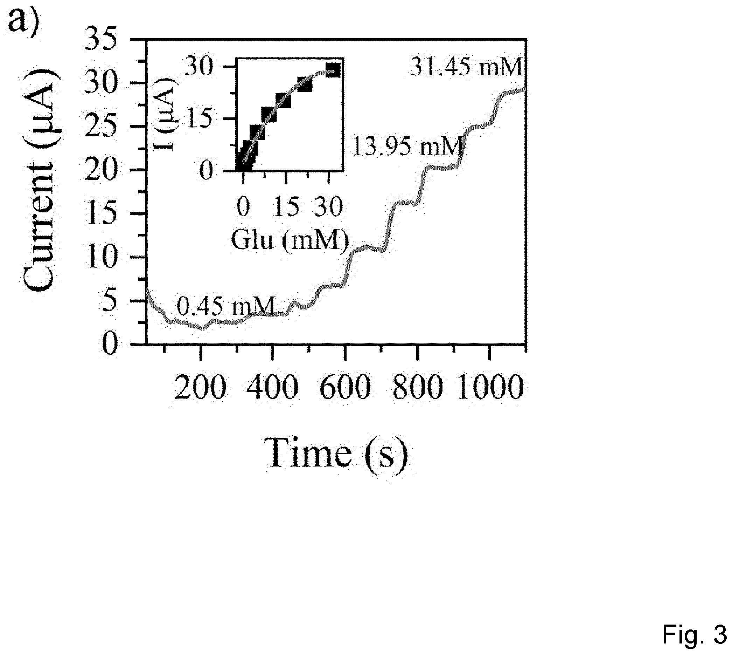

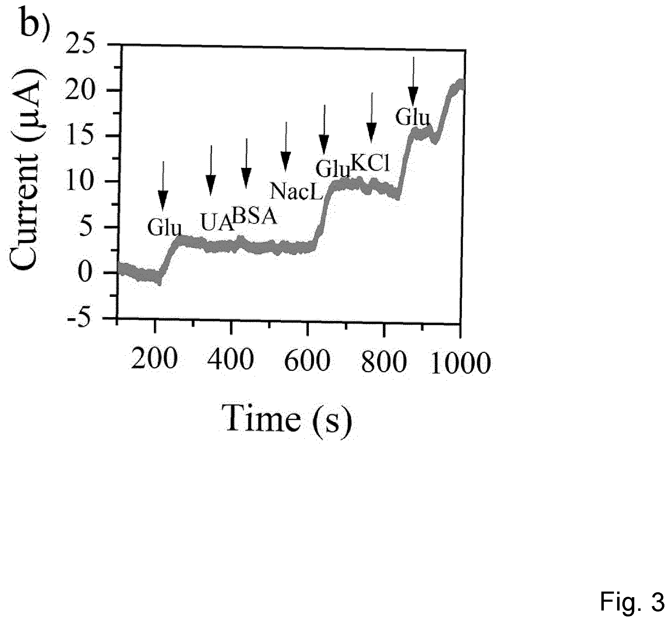

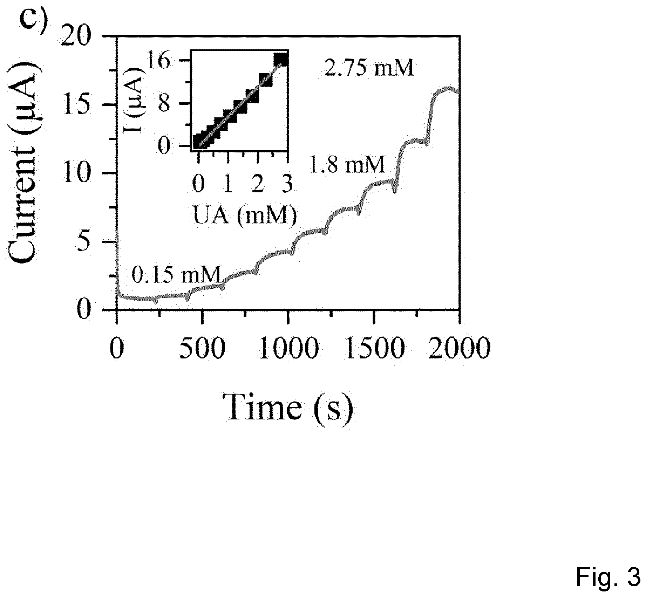

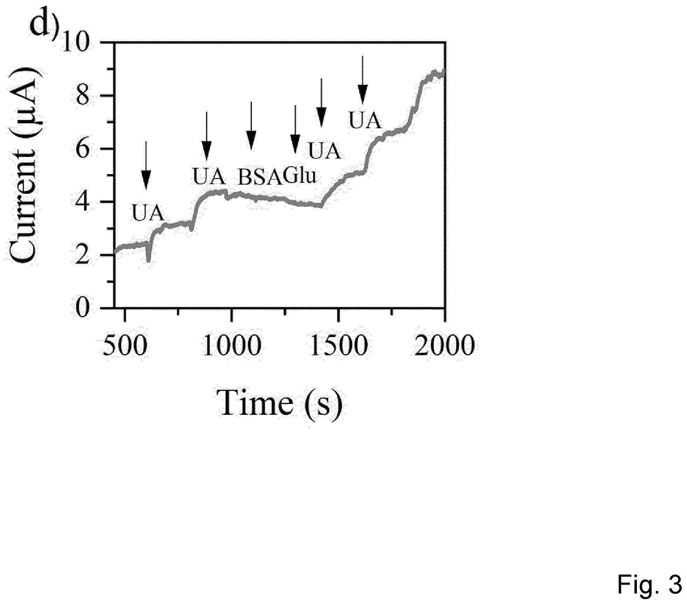

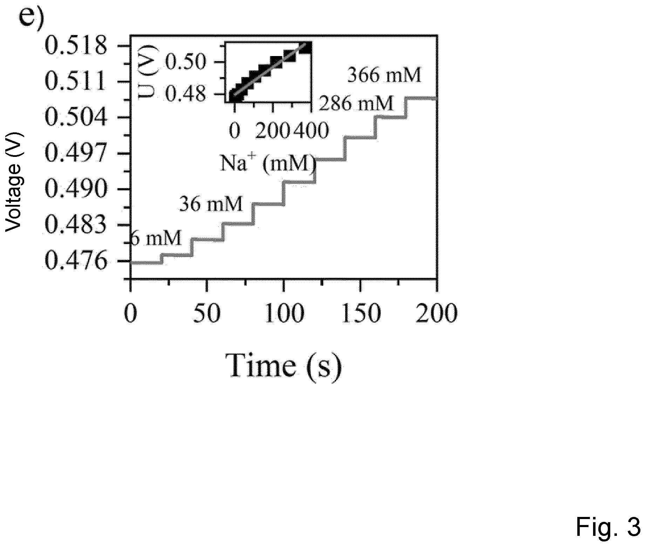

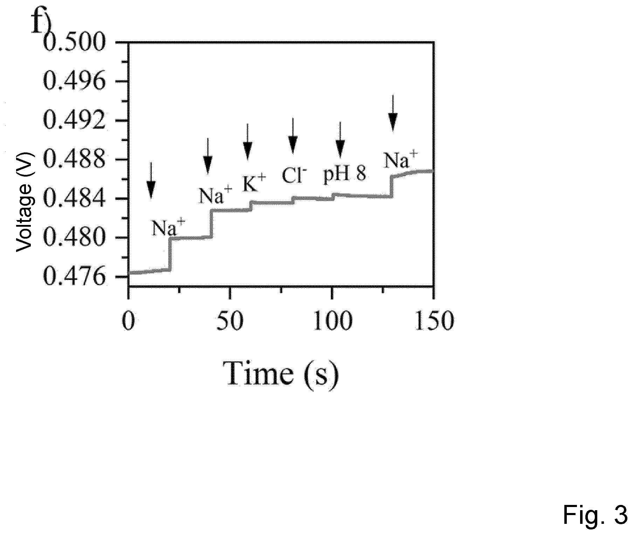

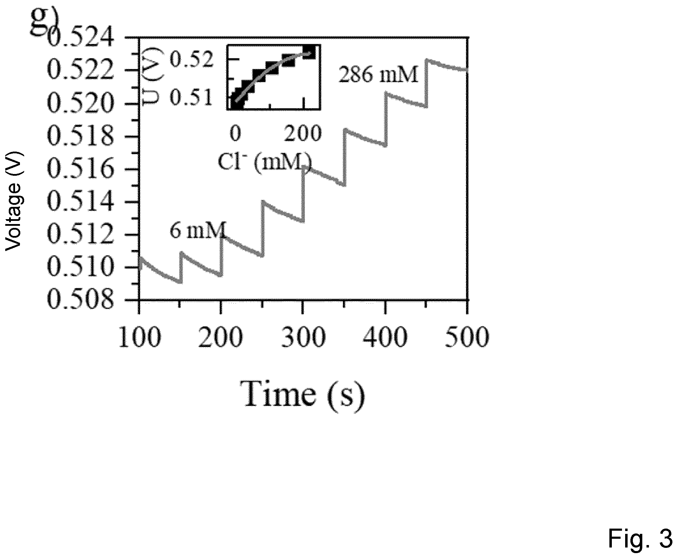

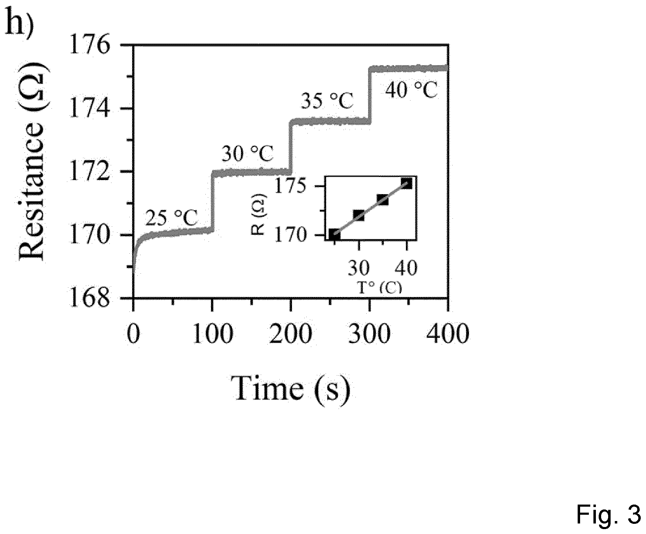

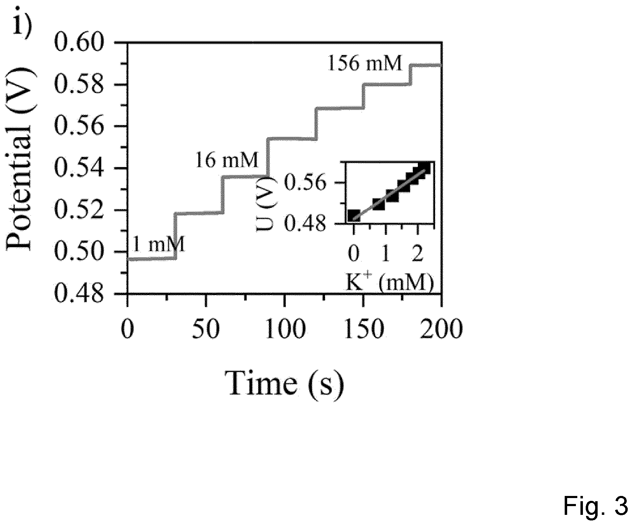

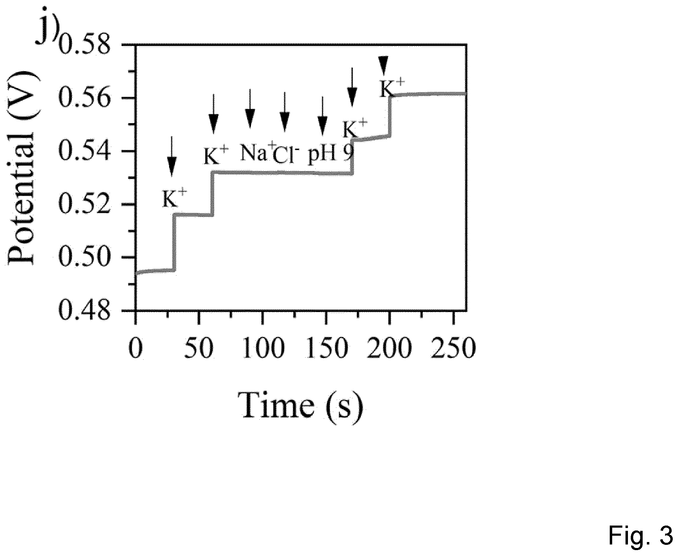

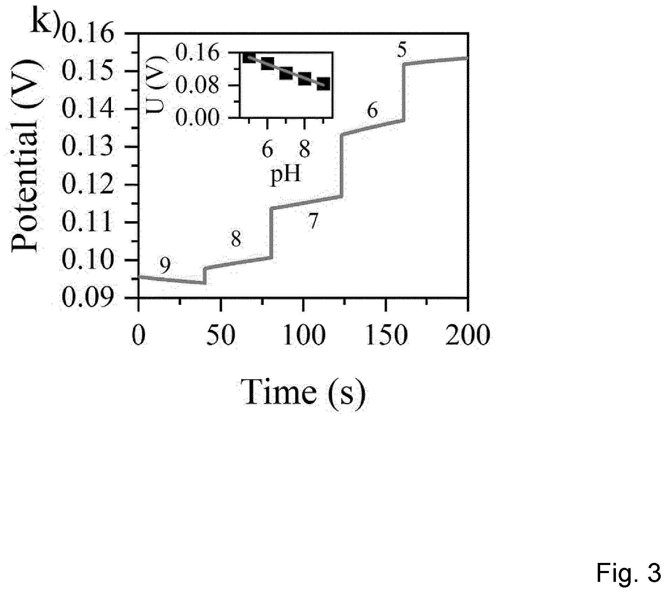

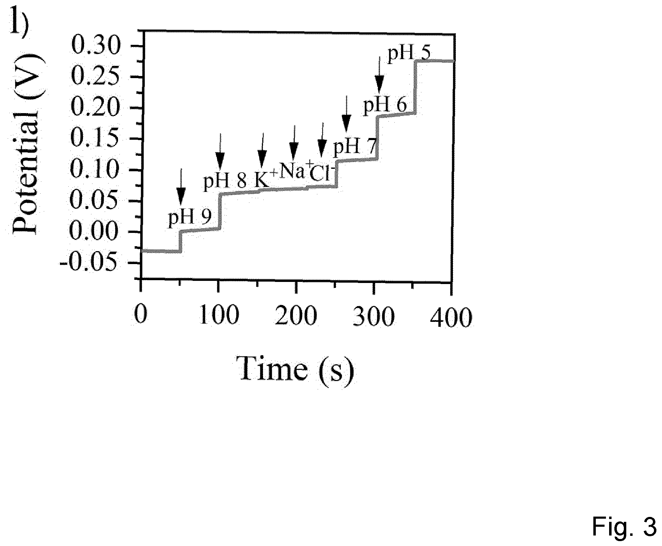

Figure 3 shows the design and characterization of the sensor array for multiplexed wound analysis in wound based ISF. a-d) Amperometric responses and selectivity of Glu and UA. Insets in a) and c), the calibration plots with a linear fit. e-g) Potentiometric response of Na⁺ and Cl⁻ and selectivity of Na⁺. Insets in e) and g), the calibration plot with a linear fit. h) Resistive response of an Pt-based T sensor under temperature changes in physiologically relevant range. Insets, the calibration plot with a linear fit. i-l) Potentiometric response and selectivity of K⁺ and polyaniline-based pH sensor. Insets in i) and k), the calibration plot with a linear fit.

The amperometric current signals generated from the PU-coated enzymatic Glu and UA sensors are proportional to the physiologically relevant concentrations of the corresponding metabolites in ISF, with sensitivities of 12 and 80 µAmM⁻¹cm⁻², respectively, and the sensors also exhibited excellent selectivity. For Na⁺, Cl⁻, K⁺ sensors, the response principle is based on the change in the corresponding ion-selective membrane potentials resulting from the changes in ion levels. Figure 3e, 3g, and 3i display the voltage response of the Na⁺ sensor in 1-366 mM, the Cl⁻ sensor in 1-366 mM, and the K⁺ sensor in 1-156 mM in ISF solution, respectively. All three ion-selective electrodes exhibited good linearity with average sensitivities of 48 mV (R² = 0.98) for Na⁺, 41 mV (R² = 0.96) for Cl⁻, and 32 mV (R² = 0.9996) for K⁺ per decade of concentration, which are also close to the slope of the Nernst equation. All the ion sensors also exhibited excellent selectivity against different interferents (Figures 3f and 3j). An integrated Pt-based resistive T sensor is part of the sensor array. It has a sensitivity of about 0.35% °C⁻¹ in the physiological temperature range of 25° to 40°C (Figure 3h). Similarly, the pH sensor uses an electrodeposited polyaniline film as the pH-sensitive membrane and showed a good sensitivity of 45.7 mV/pH and selectivity (Figures 3k and 3l).

### Correlation between ISF and wound exudate:

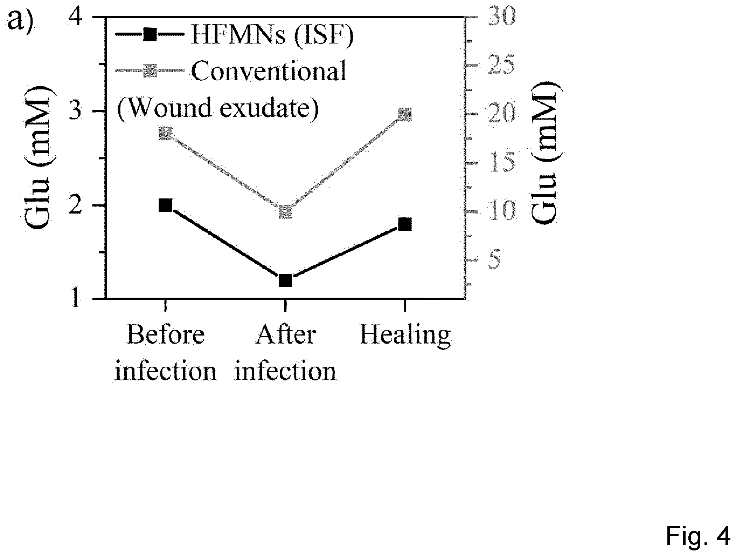

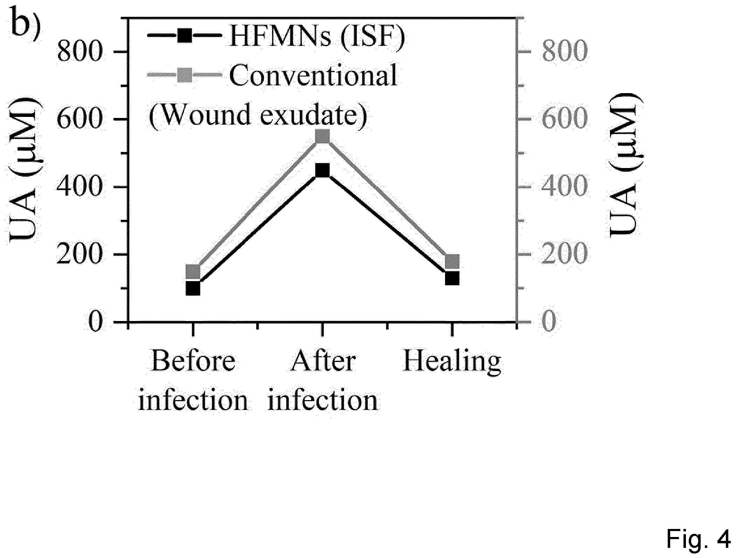

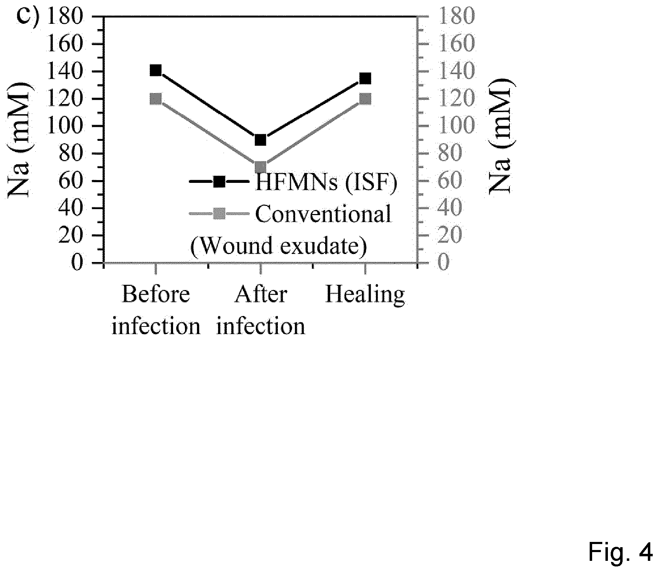

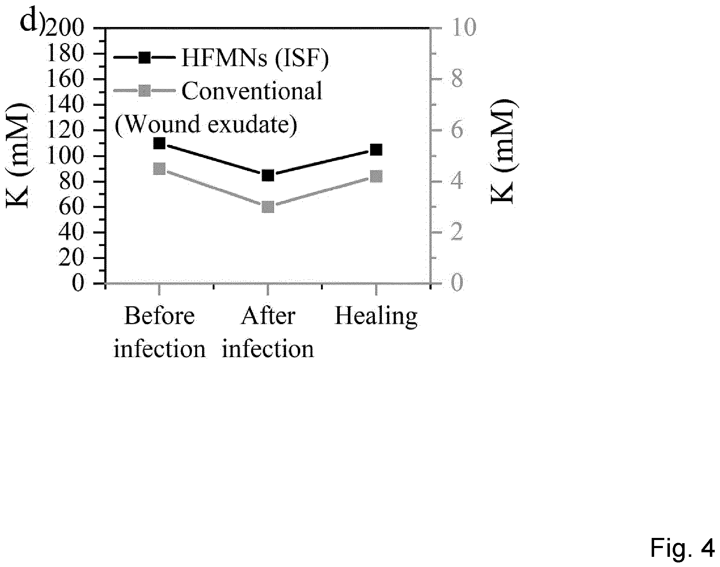

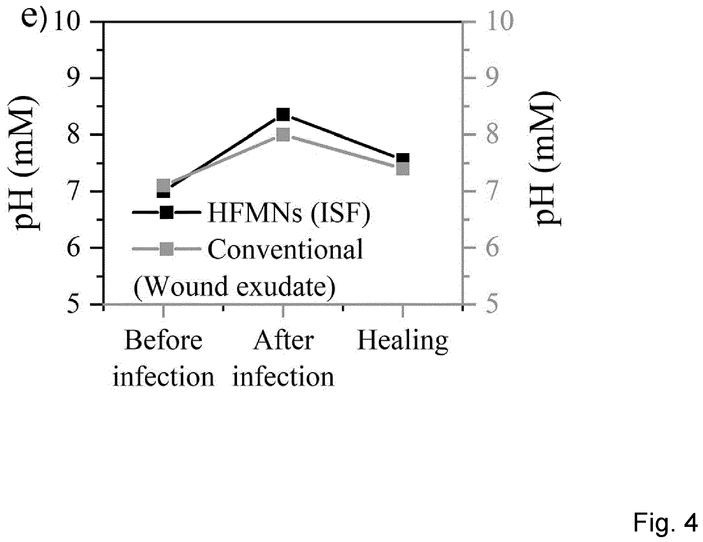

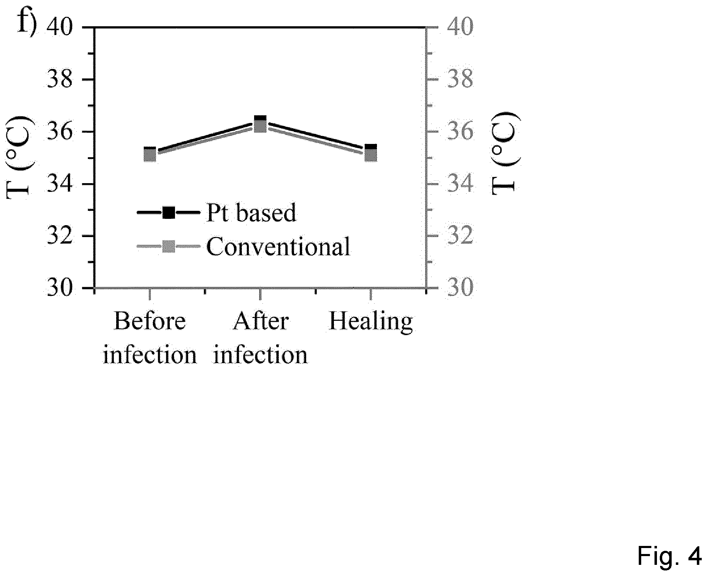

Figure 4 shows the correlation of the HFMNs system and conventional sensors for multiplexed wound biomarker monitoring in wound ISF and exudate, respectively, in three different ex vivo systems (before infection, after infection, and healing). a-g) Glu, UA, Na⁺, Cl⁻, T, K⁺, and pH sensing.

The biomarkers, as earlier measured by the HFMNs-based implantable system, were further independently assessed using conventional approaches in artificial wound exudate to establish the correlation and validation of the developed implantable system. The results of monitoring each indicator in three different states (before infection, after infection, and healing state) in CW patients ISF and wound exudate by adding and mimicking the exact states are illustrated in Figure 4a-f. Substantially, elevated UA, T, pH, levels were observed as compared to those before infection⁴. The increase in temperature can be potentially linked to inflammation¹⁵. The elevated levels of UA after infection can be due to up-regulation of xanthine oxidase, a component of the innate immune system responding to inflammatory cytokines in chronic ulcers that plays a key role in purine metabolism to produce UA¹⁶. pH and UA are acidity related, and their elevation during the bacteria infection has also been widely reported¹⁷. In contrast, the Glu level in infected wound fluid showed >36% decrease after infection, attributing to the increased glucose consumption of bacteria activities¹⁸. Upon wound treatment, the T, pH, and UA decreased toward the levels before the infection, while the Glu level increased significantly after treatment, indicating the successful bacterial elimination. Correspondingly, relatively high levels of Na⁺ and K⁺ were monitored in the early stages of infection. Subsequently, the levels of Na⁺ and K⁺ in the wound exudate gradually decreased and again exhibited an increasing trend during the healing, which in the case of normal wound could be explained by the demand for more abundant ions level involvement in wound healing, while for infected wound could be attributed to bacterial proliferation. These measurements in ISF also exhibited similar trends of artificial wound fluid, demonstrating that the HFMNs is able to report objective quantitative data within clinically relevant ranges.

### Characterization of the therapeutic capabilities

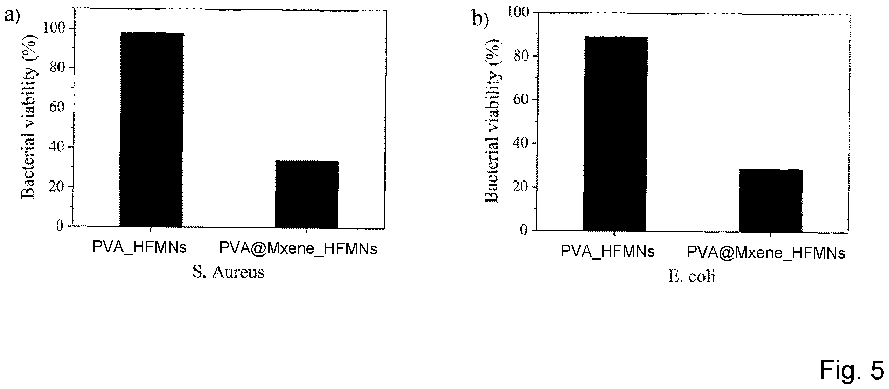

Figure 5 shows the antimicrobial activity of the hydrogel of PVA and LSPS based PVA/MXene HFMNs. a) S. Aureus. b) E. coli.

The ideal wound dressing should have excellent antibacterial activity. The antibacterial activity of the HFMNs-based implantable dressing against S. aureus and E. coli was detected using the bacterial counting method. As shown in Figures 5a and 5b, S. aureus and E. coli were incubated with the both PVA/Ch and LSPS-based PVA/Ch/MXene HFMNs at 37 °C for 6 h, with a large number of colonies. Owing to the damage caused by MXene to bacterial cell membrane, the number of bacteria in final HFMNs was lower than that in the PVA group¹⁹. Meanwhile, the inhibition rates of the LSPS-based PVA/Ch/MXene hydrogel against E. coli and S. aureus were more than 90%, indicating that it had excellent antibacterial activity.

In the following, further embodiments of the invention are described.

### Design and fabrication strategies of the fully-integrated HFMNs-based implantable dressing system

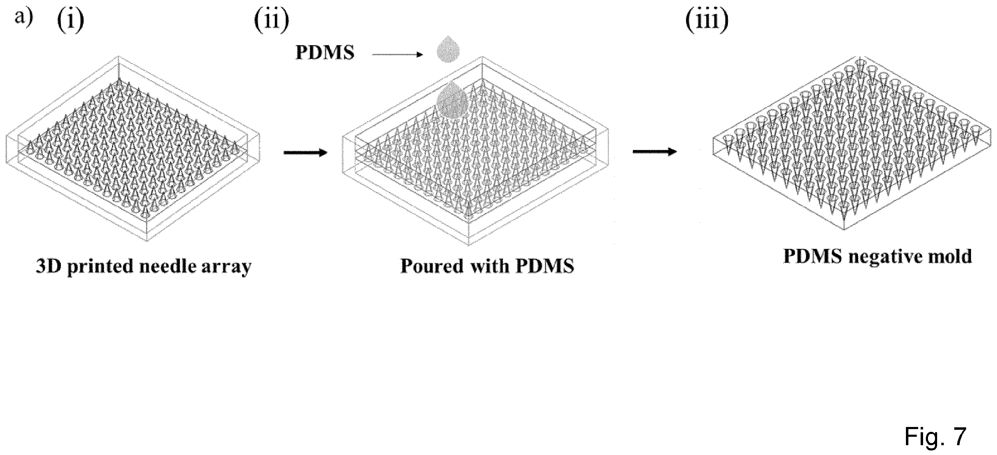

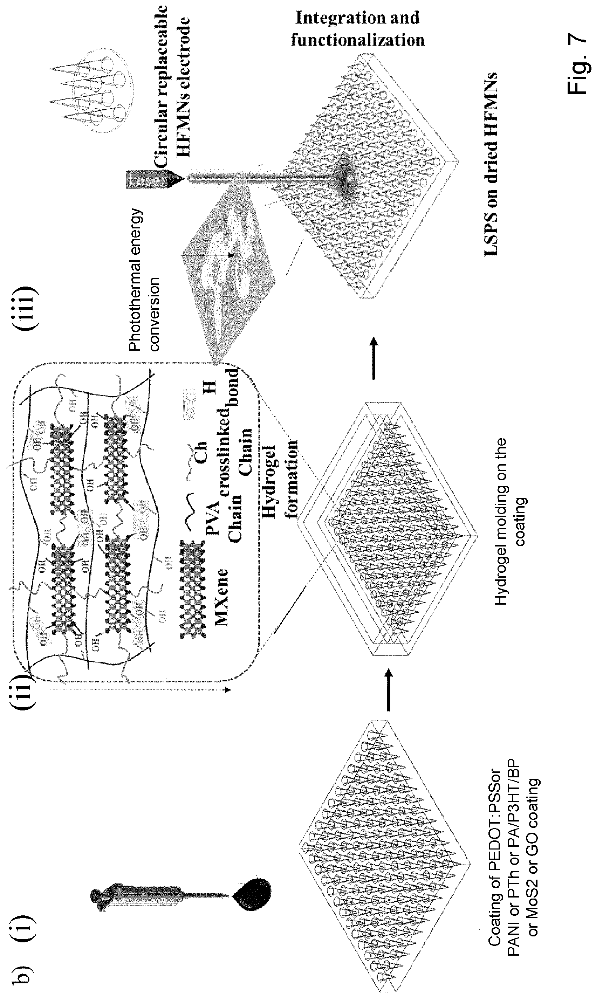

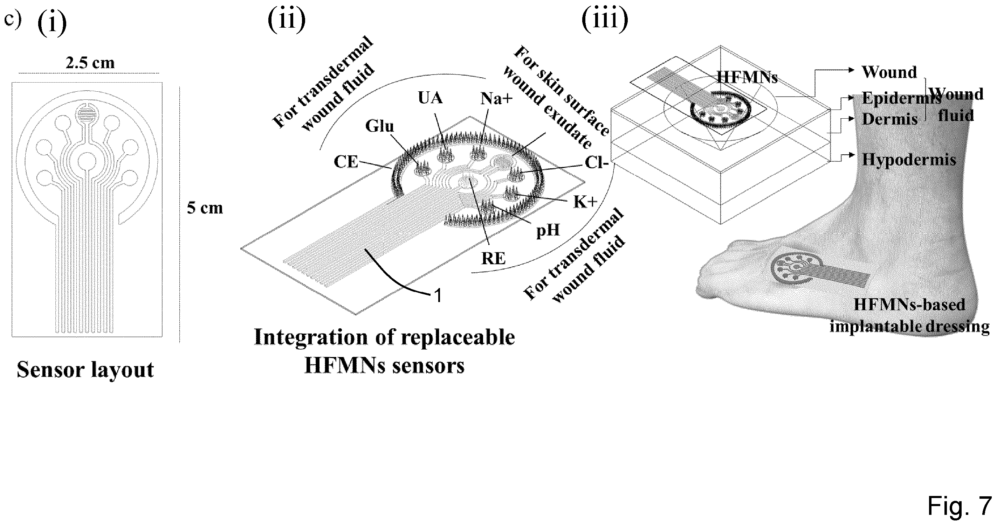

Figure 7 shows another fabrication concept of an HFMNs-based implantable dressing system for CWs diagnostics and therapy. The fabrication process in step a) involves designing the HFMNs and their 3D printing. Step b) includes coating of conductive polymers, hydrogel formation, and subsequent LSPS process, Step c) includes sensor layout (CE-counter electrode and RE-reference electrode), integration and functionalization of the replaceable sensors onto the sensor pad, and placement of the HFMN-based implantable system for CWs monitoring and therapy.

Herein, solid microneedles consisting of 49×43 conically-shaped needles (800 µm in height) arranged atop a 60×40 mm film (5mm in thickness) were produced via stereolithography 3D printing (Figure 7a (i)) using a computer-aided design (CAD) and used as the master mold for the generation of polydimethylsiloxane (PDMS) negative molds (Figure 7a (ii) and (iii)).A electroactive layer of conductive polymer mixture, comprising either (PEDOT: PSS, PANI, PTh, or PA, combined with P3HT, further integrated with light-responsive two-dimensional materials selected from BP, MoS₂, and GO drop casted onto the negative mold (Figure 7b (i)), followed by hydrogel casting on the PDMS molds (Figure 7b (ii)). After two times of freezing and thawing, the HFMNs were then released from PDMS, dried under vacuum, and utilized for further modifications. Opting to use 3D printing for microneedle development can provide a scalable, consistently reproducible, and significantly cheaper manufacturing approach. The hydrogel was made of polyvinyl alcohol (PVA), chitosan (Ch), and MXene (Ti₃C₂Tₓ), in which the biocompatible PVA can form a 3D network with Ch via cross-linking by repeated freezing and thawing (Figure 7 b (ii)), and Ch can improve the porosity and water absorption capacity of hydrogel⁷. The phase transition property and 3D network structure of the PVA/Ch hydrogel make the microneedle suitable for both easy penetration into skin and the extraction of sufficient wound fluid and ISF. MXene has metallic conductivity and a larger specific surface area, as well as an improved electronegative functional group (-OH, -F, -O)⁸. In the presence of electronegative groups on Ti₃C₂Tₓ, 3D polymer hydrogel networks form through the hydrogen (H) bonds and enhance the mechanical and electrical properties of the hydrogel (see the hydrogel formation in Figure 7b(ii)).

### Laser-scribed phase separation (LSPS)-based photothermal conversion

To exploit this technology, we utilized a photothermal method in conjunction with light-responsive two-dimensional materials, specifically selected for their compatibility with laser patterning processes. This approach allows for the creation of highly conductive hydrogels directly on microneedles without necessitating a subsequent chemical cleaning process, as illustrated in Figure 7b (iii). PEDOT: PSS exhibits a phase dispersion in which the insulating PSS is located in the shell and the conductive PEDOT is located in the core. The PSS shell around the PEDOT core not only obstructs charge flow but also plays a role in PEDOT dispersion⁹. A key approach for converting PEDOT: PSS into hydrogels that are water-stable is to restructure the phase organization of the material. Phase separation is a novel method for producing conductive PEDOT: PSS hydrogels by careful alteration of the orientation and crystallization of the PEDOT and PSS-rich regions, respectively^{10,11}. Precisely, outstanding electrical conductivity and aqueous stability can be achieved through a robust interconnection between the hydrophobic and conductive regions rich in PEDOT. Therefore, we employed the CO₂ laser patterning process to fabricate highly conductive hydrogels via the LSPS without the need for a separate postchemical cleaning step on PEDOT: PSS/GO coated HFMNs (Figure 7b (iii)). In order to optimize laser absorption, PEDOT: PSS was sonicated with GO solution with varying concentrations as the solvent. This process facilitated the separation and recrystallization of PEDOT and PSS via a fast temperature increase and an enhanced electric field. We proposed that any laser is capable of simultaneously supplying photothermal energy and an electric field to PEDOT: PSS/GO, in light of the improved properties of PEDOT: PSS-based hydrogels.

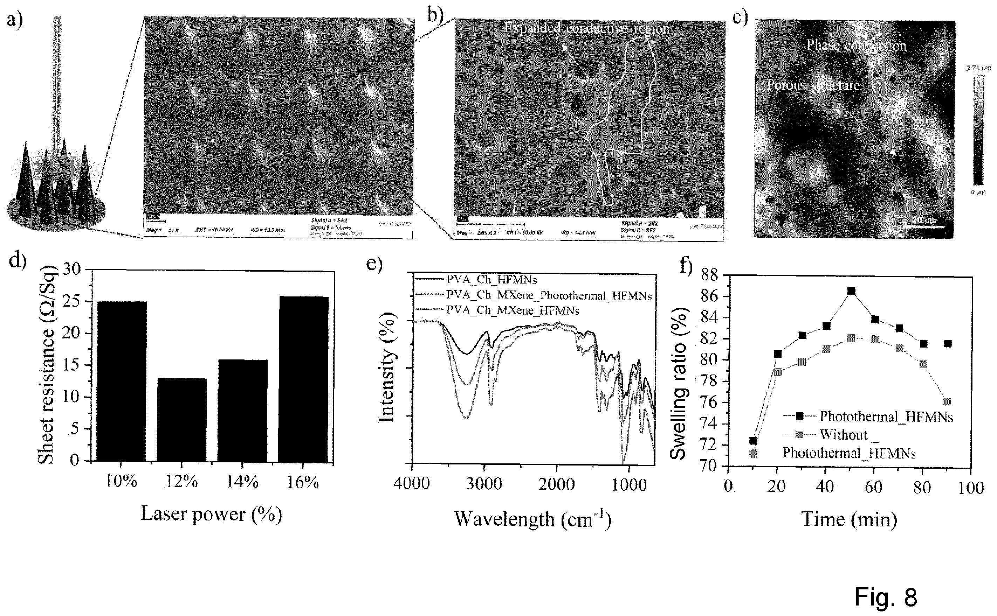

Figure 8 shows physical characterizations of the HFMNs. a) SEM image of the laser-scribed HFMNs and b) expanded porous PEDOT rich region due to LSPS. c) AFM phase image of LSPS. d) Laser power optimization for LSPS. e) FTIR spectra analysis. f) Swelling ratio.

As shown by scanning electron microscopy (SEM) (Figure 8a), HFMNs exhibited a conical structure with a length of -P800 µm, a sharp tip of -P10 µm, and inter-microneedle spacing of 700 µm after the LSPS technique. This emphasizes the homogenous microstructural characteristics of the coated laser-scribed microneedles. Selective phase separation by the laser was confirmed with SEM and atomic force microscopy (AFM) phase image analysis (Figure 8b and 8c). The PEDOT-rich domain was greatly expanded (bright color) and connected after the LSPS. When the laser was working properly, increasing the laser power (LP) made PEDOT: PSS more electrically conductive and stable in water. But when the laser power was too high, PEDOT: PSS turned into carbon and its conductivity started to drop (Figure 8d). For LP 12%, combined with the effect of GO, fast laser scanning (300 s) can achieve the highest electrical conductivity (sheet resistance of 13 Ω/Sq). After the LSPS, the desired circular-shape electrodes can be cut and utilized for replaceable sensors for CWs monitoring and treatment in the dermis and/or epidermis level (Figure 7c (ii) and (iii)). This novel manufacturing process ensures the fabrication and mass production of conductive HFMNs at a fast-processing speed compared with other processes.

The Fourier transform infrared (FT-IR) spectra of the PVA/Ch, PVA/Ch/MXene, and LSPS-based PVA/Ch/MXene HFMNs (Figure 8e) showed peaks at 3417, 2927, 1616, 1413, and 1029 cm⁻¹, which correspond to the O-H stretching vibration, C-H stretching vibration, COO-stretching vibration, carbonyl (C=O) stretching vibration, and C-O-C stretching vibration¹². By comparison, it seems that after LSPS, the peak intensity of the HFMNs decreased slightly due to the increase in hydrophobic PEDOT-rich regions.

As shown in Figure 8f, the swelling rate and water content of the HFMNs and LSPS-based HFMNs were compared. The results showed that the LSPS technique increased its swelling rate and water stability. The increased swelling rate can make the gel more effective at absorbing transdermal wound fluid or ISF and wound healing.

### Influence of photothermal techniques on electrochemical signaling

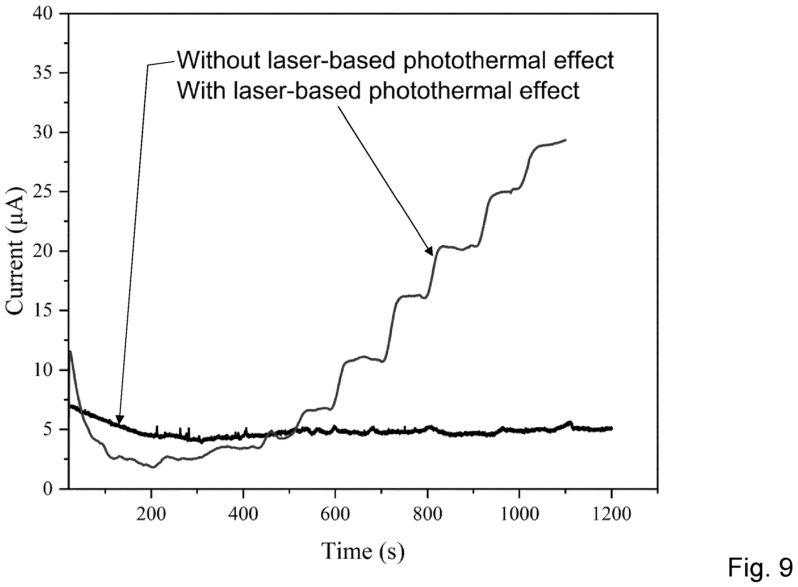

The adoption of phase conversion via photothermal techniques is crucial in enhancing the ability of microneedles to transduce electrochemical signals effectively. The graph, denoted as Figure 9, offers a comparative illustration of the electrochemical response of microneedles to glucose, with and without the application of a laser-based photothermal effect.

In the absence of the photothermal effect, the amperometric response to glucose exhibits a relatively flat line, indicating minimal to no current change over time. This suggests that without the laser treatment, the microneedles' hydrogel does not achieve the phase changes necessary for significant electrochemical signaling, thus rendering it ineffective for glucose detection.

Conversely, the red curve representing microneedles subjected to laser treatment depicts a pronounced current response that correlates directly with specific glucose concentrations. This enhanced response is indicative of the successful transformation of the hydrogel's properties brought about by the photothermal effect. The laser treatment initiates a phase separation process, which, in turn, leads to the formation of conductive pathways within the hydrogel, thereby elevating its ability to conduct electricity.

The photothermal conversion technique, therefore, not only augments the electrical conductivity but also reinforces the aqueous stability of the hydrogel, ensuring reliable performance in a biological environment. This technological advancement can be pivotal for the development of responsive and precise biosensors in medical devices, particularly those employed in the monitoring and management of biomarkers in chronic wound patients.

### Design and characterization of the HFMNs sensor array for multiplexed CW-biomarker analysis

For unavoidable inactivation of enzymes and mechanical friction, we utilized a replaceable sensor component as an effective strategy to prolong the service life of the implantable systems. A unique polar-type sensor array of 7 working electrodes was fabricated on a flexible substrate (2.5×5 cm) with their leads insulated with a waterproof layer of PDMS (Figure 7c (i)). All active circular laser-cutter HFMNs-based sensors (Glu, UA, pH, Na⁺, Cl⁻, K⁺, and T) along with the counter electrode (CE) and reference electrode (RE) are placed in independent and replaceable modules (Figure 7c (ii)). Therefore, if an electrode is used, expired, or damaged, a replacement "insert" can simply be dropped into place in the sensor platform. Moreover, it will be easier to switch the production line from one analyte to the next in a manufacturing environment. The electrode pad was brush-painted with a thin layer of chitosan as the liquid adhesive and then coupled with a patch of HFMNs. The performance of the sensor was characterized by artificial wound fluid and ISF. For the first time, we developed this novel strategy for the fabrication of HFMNs-based implantable dressing systems. The concentrations of key markers in wound exudate (WX) and ISF could generally be considered as (Glu: ISF (0-7) mM and WX (0-28) mM, UA: ISF (200-600) µM and WX (0-760) µM, Na⁺ and CI-: ISF: (0.75-200) mM and WX (5-160) mM, K⁺: ISF (1-128) mM and WX (3.2-5.7) mM, pH: ISF (5-9) and WX (4-9), and T: (25-40) °C ^{13,14.} Therefore, the concentration range of the corresponding markers in ISF is larger than the wound exudate range, which pose another major challenge to obtain linear sensor response in the physiological concentration ranges. To address these issues and achieve accurate wound fluid metabolic monitoring, increase sensor range, and minimize biofouling effects, we explored the use novel functionalization process and an outer porous membrane that serves as a diffusion limiting layer to protect the enzyme, tune response, increase operational stability, as well as enhance the linearity and sensitivity magnitude of the sensor. We fabricated our enzymatic Glu and UA sensor based on Platinum nanoparticles/ EDC-NHS/Glucose oxidase or Uricase (Pt NPs/ EDC-NHS /GOx or UOx) with additional porous membrane coatings with polyurethane (PU). The triple coating of PU-based enzymatic sensors showed the highest linearity over the wide physiological concentration range as well as high reproducibility in complex wound fluid matrix.

### Correlation between ISF and wound exudate:

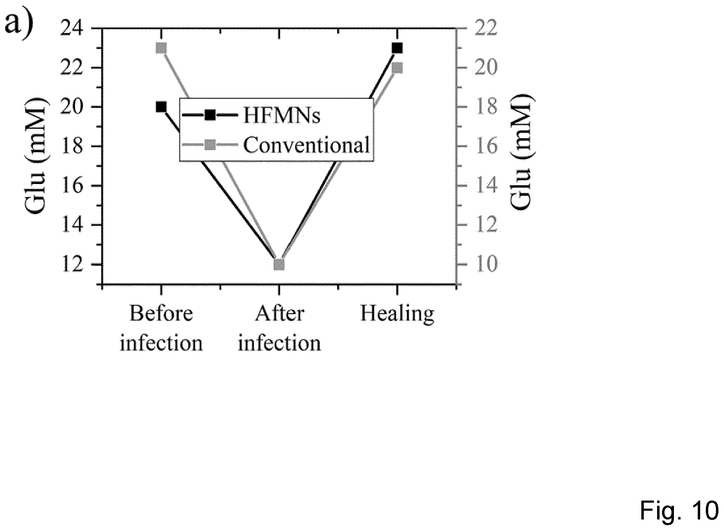

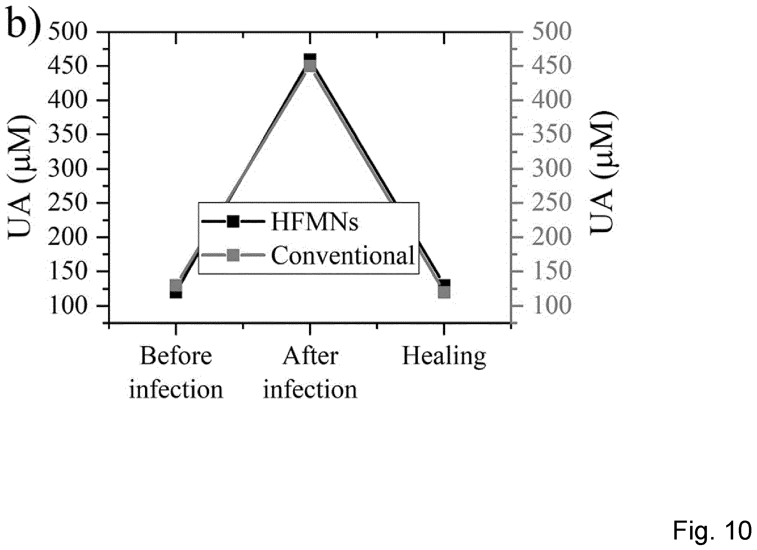

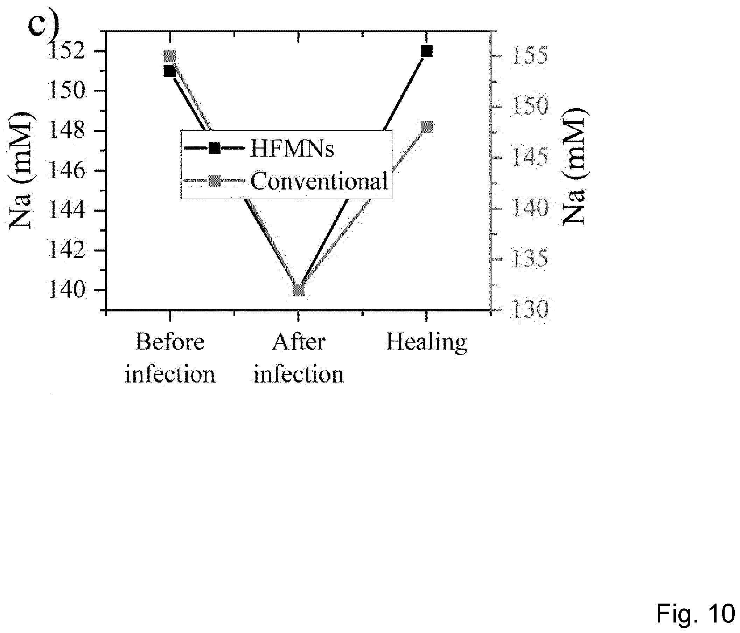

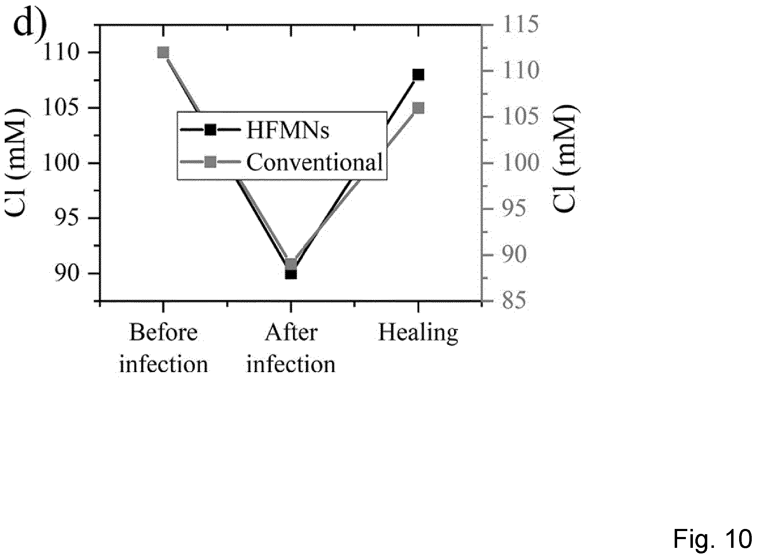

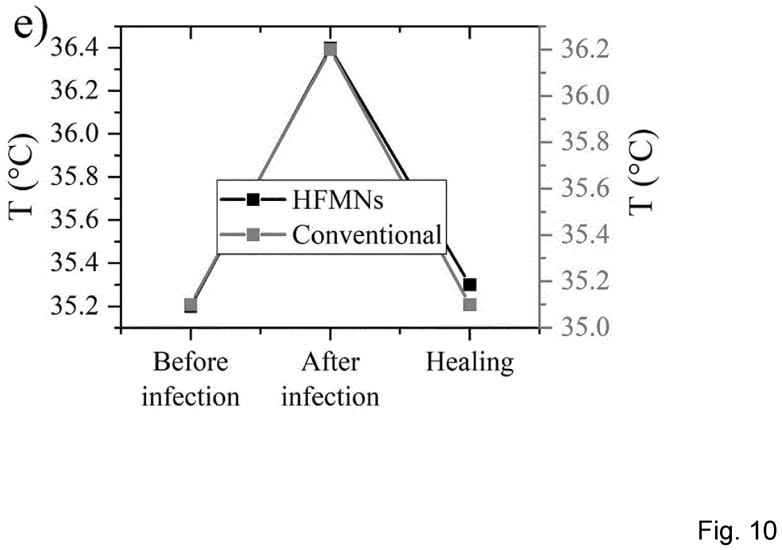

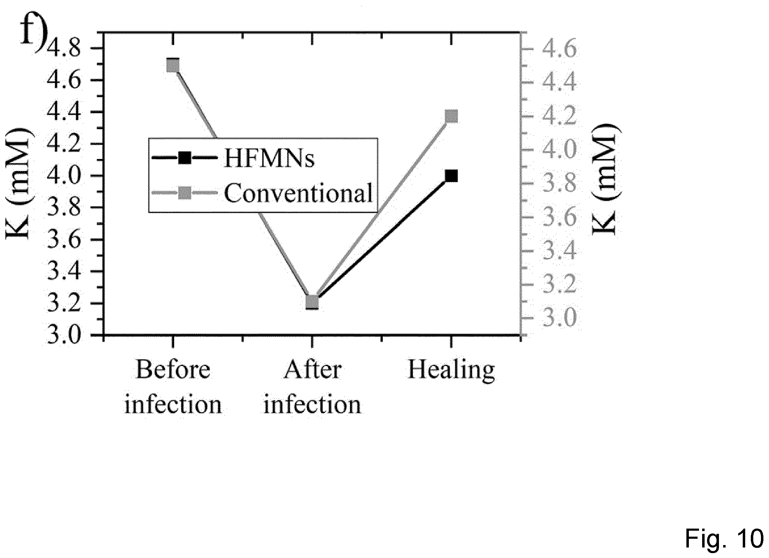

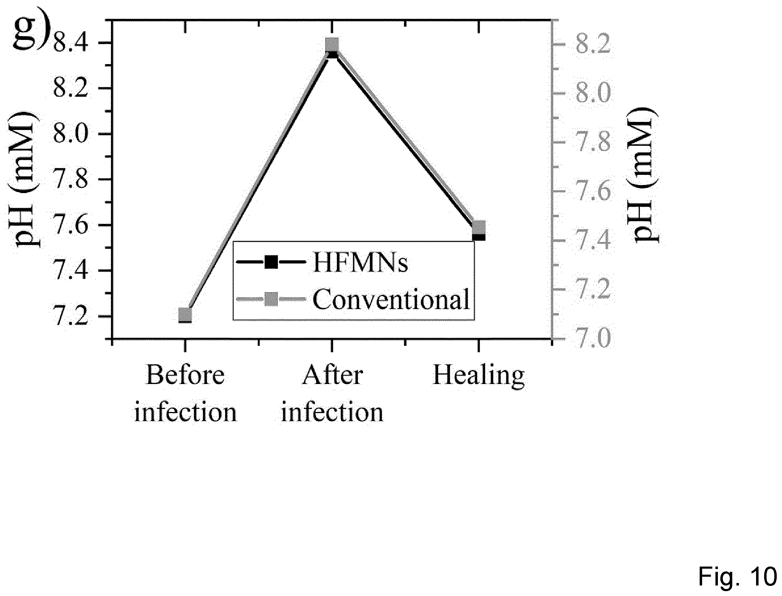

Figure 10 shows the correlation of the HFMNs system and conventional sensors for multiplexed wound biomarker monitoring in wound ISF and exudate, respectively, in three different ex vivo systems (before infection, after infection, and healing). a-g) Glu, UA, Na⁺, Cl⁻, T, K⁺, and pH sensing.

The biomarkers, as earlier measured by the HFMNs-based implantable system, were further independently assessed using conventional approaches in artificial wound exudate to establish the correlation and validation of the developed implantable system. The results of monitoring each indicator in three different states (before infection, after infection, and healing state) in CW patients ISF and wound exudate by adding and mimicking the exact states are illustrated in Figure 10a-f. Substantially, elevated UA, T, pH, levels were observed as compared to those before infection⁴. The increase in temperature can be potentially linked to inflammation¹⁵. The elevated levels of UA after infection can be due to up-regulation of xanthine oxidase, a component of the innate immune system responding to inflammatory cytokines in chronic ulcers that plays a key role in purine metabolism to produce UA¹⁶. pH and UA are acidity related, and their elevation during the bacteria infection has also been widely reported¹⁷. In contrast, the Glu level in infected wound fluid showed >36% decrease after infection, attributing to the increased Glu consumption of bacteria activities¹⁸. Upon wound treatment, the T, pH, and UA decreased toward the levels before the infection, while the Glu level increased significantly after treatment, indicating the successful bacterial elimination. Correspondingly, relatively high levels of Na⁺ and K⁺ were monitored in the early stages of infection. Subsequently, the levels of Na⁺ and K⁺ in the wound exudate gradually decreased and again exhibited an increasing trend during the healing, which in the case of normal wound could be explained by the demand for more abundant ions level involvement in wound healing, while for infected wound could be attributed to bacterial proliferation. These measurements in ISF also exhibited similar trends of artificial wound fluid, demonstrating that the HFMNs is able to report objective quantitative data within clinically relevant ranges.

### Characterization of the therapeutic capabilities

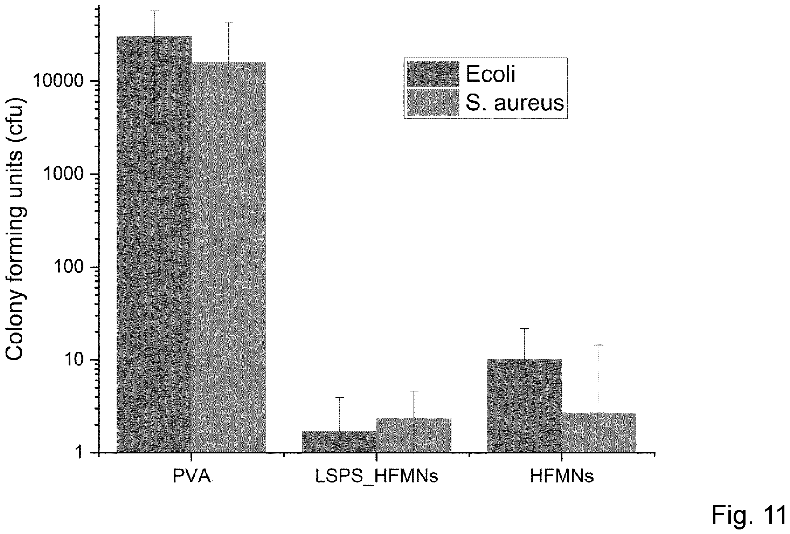

Figure 11 shows the antimicrobial activity of the hydrogel of PVA and LSPS based PVA/MXene HFMNs against S. Aureus and E. coli with error bars.

The ideal wound dressing should have excellent antibacterial activity. The antibacterial activity of the HFMNs-based implantable dressing against S. aureus and E. coli was detected using the bacterial counting method (Figure 11)., S. aureus and E. coli were incubated with the both PVA/Ch and LSPS-based PVA/Ch/MXene HFMNs at 37 °C for 6 h, with a large number of colonies. Owing to the damage caused by MXene to bacterial cell membrane, the number of bacteria in final HFMNs was lower than that in the PVA group¹⁹. Meanwhile, the inhibition rates of the LSPS-based PVA/Ch/MXene hydrogel against E. coli and S. aureus were more than 90%, indicating that it had excellent antibacterial activity.

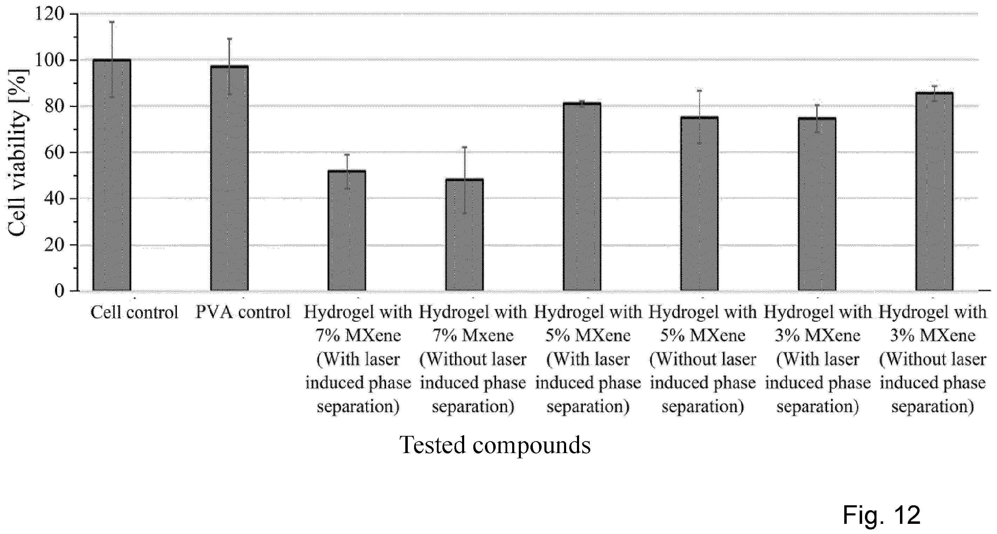

Figure 12 shows cell viability of fibroblasts after 7 days with hydrogels containing 7%, 5%, or 3% MXene, with and without LSPS.

The cytotoxicity assay results highlight the performance of hydrogels containing varying concentrations of MXene, both with and without LSPS, in supporting cell viability and antimicrobial activity. Primary human fibroblasts were cultured for seven days with the hydrogels placed in transwell inserts, and cell viability was measured using the WST-8 assay. The findings indicate that hydrogels with 7% MXene significantly reduce cell viability, particularly when prepared without LSPS, due to the strong antimicrobial effects of MXene at this concentration, which can negatively impact fibroblast health. However, applying LSPS improves cell compatibility to some extent by refining the hydrogel structure. Hydrogels with 5% MXene achieve the most balanced performance, demonstrating high cell viability alongside effective antimicrobial activity. LSPS further enhances their performance by reducing cytotoxic effects and improving uniformity, making this formulation ideal for chronic wound applications where both infection control and tissue regeneration are critical. In contrast, hydrogels with 3% MXene exhibit excellent biocompatibility, with cell viability comparable to controls, but their lower MXene content compromises antimicrobial efficacy, limiting their suitability for infection-prone wounds. These findings underscore the importance of both MXene concentration and LSPS in optimizing the hydrogel's properties for chronic wound care. The 5% MXene hydrogel with LSPS represents an innovative solution, balancing biocompatibility and antimicrobial functionality to provide superior wound-healing capabilities. This unique combination leverages the antimicrobial potency of MXene and the structural refinement from LSPS to address the dual challenges of infection control and tissue regeneration, making it a promising approach for advanced wound dressing systems.

### References

1. Sharifuzzaman, M. et al. Smart bandage with integrated multifunctional sensors based on MXene-functionalized porous graphene scaffold for chronic wound care management. Biosens. Bioelectron. 169, 112637 (2020).
2. Dong, R. & Guo, B. Smart wound dressings for wound healing. Nano Today at https://doi.orq/10.1016/i.nantod.2021.101290 (2021).
3. Mostafalu, P. et al. Smart Bandage for Monitoring and Treatment of Chronic Wounds. Small (2018) doi:10.1002/smll.201703509.
4. Shirzaei Sani, E. et al. A stretchable wireless wearable bioelectronic system for multiplexed monitoring and combination treatment of infected chronic wounds. Sci. Adv. (2023) doi:10.1126/sciadv.adf7388.
5. Kiang, T., Ranamukhaarachchi, S. & Ensom, M. Revolutionizing Therapeutic Drug Monitoring with the Use of Interstitial Fluid and Microneedles Technology. Pharmaceutics 9, 43 (2017).
6. Scallan, J., Huxley, V. H. & Korthuis, R. J. Capillary Fluid Exchange: Regulation, Functions, and Pathology. Colloq. Ser. Integr. Syst. Physiol. From Mol. to Funct. (2010) doi:10.4199/c00006ed1v01y201002isp003.
7. He, R. et al. A Hydrogel Microneedle Patch for Point-of-Care Testing Based on Skin Interstitial Fluid. Adv. Healthc. Mater. 9, (2020).
8. Alhabeb, M. et al. Guidelines for Synthesis and Processing of Two-Dimensional Titanium Carbide (Ti3C2Tx MXene). Chem. Mater. (2017) doi:10.1021/acs.chemmater.7b02847.
9. Kim, S. M. et al. Influence of PEDOT:PSS crystallinity and composition on electrochemical transistor performance and long-term stability. Nat. Commun. (2018) doi:10.1038/s41467-018-06084-6.
10. Yao, B. et al. Ultrahigh-Conductivity Polymer Hydrogels with Arbitrary Structures. Adv. Mater. (2017) doi:10.1002/adma.201700974.
11. Won, D. et al. Digital selective transformation and patterning of highly conductive hydrogel bioelectronics by laser-induced phase separation. Sci. Adv. (2022) doi:10.1126/sciadv.abo3209.
12. Liu, H. et al. Approaching intrinsic dynamics of MXenes hybrid hydrogel for 3D printed multimodal intelligent devices with ultrahigh superelasticity and temperature sensitivity. Nat. Commun. (2022) doi:10.1038/s41467-022-31051-7.
13. Gao, Y. et al. A flexible multiplexed immunosensor for point-of-care in situ wound monitoring. Sci. Adv. 7, (2021).
14. Liu, Z. et al. Integrated Multiplex Sensing Bandage for in Situ Monitoring of Early Infected Wounds. ACS Sensors (2021) doi:10.1021/acssensors.1c01279.
15. Chanmugam, A. et al. Relative Temperature Maximum in Wound Infection and Inflammation as Compared with a Control Subject Using Long-Wave Infrared Thermography. Adv. Skin Wound Care 30, 406-414 (2017).
16. Fernandez, M. L., Upton, Z., Edwards, H., Finlayson, K. & Shooter, G. K. Elevated uric acid correlates with wound severity. Int. Wound J. (2012) doi:10.1111/j.1742-481X.2011.00870.x.
17. Tegl, G., Schiffer, D., Sigl, E., Heinzle, A. & Guebitz, G. M. Biomarkers for infection: enzymes microbes, and metabolites. Applied Microbiology and Biotechnology at https://doi.org/10.1007/s00253-015-6637-7 (2015).
18. Zhu, Y. et al. A Multifunctional Pro-Healing Zwitterionic Hydrogel for Simultaneous Optical Monitoring of pH and Glucose in Diabetic Wound Treatment. Adv. Funct. Mater. (2020) doi:10.1002/adfm.201905493.
19. Li, Y. et al. Muscle-inspired MXene/PVA hydrogel with high toughness and photothermal therapy for promoting bacteria-infected wound healing. Biomater. Sci. 10, 1068-1082 (2022.

## Claims

1. Microneedle array (1) comprising a flexible substrate (2) and a plurality of microneedles arranged in the form of one or more arrays which are configured as a wound dressing system, and comprising different types of sensors (4, 5, 6, 7, 8, 9, 10) configured for sensing different kinds wound-specific electrochemical bio-indicators of a living being wearing the microneedle array (1), wherein the microneedles are configured as hydrogel-forming microneedles with high electric conductivity of at least 100 S/m and water stability suitable for conducting electrical signals from the living being to the sensors (4, 5, 6, 7, 8, 9, 10), and providing for on-site healing capabilities due to the anti-inflammatory and antimicrobial properties of the hydrogel formed by the microneedles.

2. Microneedle array according to claim 1, **characterized in that** the microneedle array (1) is configured for application to a chronic wound of a living being.

3. Microneedle array according claim 2, **characterized in that** the microneedle array (1) is configured for diagnosis and/or therapy of the chronic wound in dermis and/or epidermis level of the living being.

4. Microneedle array according to any of the preceding claims, **characterized in that** the microneedle array (1) or at least the microneedles are created and/or modified by photothermal techniques separately, e.g. by a CO₂ laser-based photothermal process like laser-scribed phase separation (LSPS).

5. Microneedle array according to any of the preceding claims, **characterized in that** the microneedle array (1) is configured for measuring at least one parameter of the transdermal wound fluid or interstitial fluid (ISF) to get information about exudate of the wound to which the microneedle array (1) is applied to.

6. Microneedle array according to any of the preceding claims, **characterized in that** the microneedle array (1) is configured for minimal invasive implantation in the dermis and/or epidermis levels of the chronic wound-affected skin in a living being.

7. Microneedle array according to any of the preceding claims, **characterized in that** the microneedles are arranged in separate groups (insulas) on the substrate (2), wherein each group is separated from other groups by an area of the substrate (2) surrounding the group and having no microneedles.

8. Microneedle array according to claim 7, **characterized in that** each of the sensors (4, 5, 6, 7, 8, 9, 10) is implemented in one of the groups of microneedles.

9. Microneedle array according to any of the preceding claims, **characterized in that** the substrate (2) is an elastic substrate.

10. Microneedle array according to any of the preceding claims, **characterized in that** the microneedle array (1) or at least the microneedles are coated with a functionalizing material and thereby functionalized.

11. Microneedle array according to any of the preceding claims, **characterized in that** the electric conductivity of the hydrogel-forming microneedles can be at least 200 S/m or at least 300 S/m or at least 384 S/m.

12. Microneedle array according to any of the preceding claims, **characterized in that** one, more or all of the sensors (4, 5, 6, 7, 8, 9, 10) of the microneedle array (1) can be replaced on the substrate (2).

13. System for sensing wound-specific bio-indicators of a living being, comprising a microneedle array according to any of the preceding claims and an electronic circuit (14) which is electrically connected to the sensors (4, 5, 6, 7, 8, 9, 10) of the microneedle array (1), wherein the electronic circuit (14) is configured for evaluating, storing and/or displaying the bio-indicators sensed by the sensors (4, 5, 6, 7, 8, 9, 10).

14. Method for producing a microneedle array (1) according to any of the preceding claims, **characterized in that** the microneedle array (1) or at least the microneedles are created and/or modified by photothermal techniques separately, e.g. by a CO₂ laser-based photothermal process like LSPS, wherein the electrical conductivity of the hydrogel-forming microneedles is increased by the photothermal process.

15. Method according to claim 14, **characterized in that** by the photothermal process, the base material of the sensor (4, 5, 6, 7, 8, 9, 10) is coated with a functionalizing material and thereby functionalized.
